# EUROPEAN PATENT APPLICATION

(11) **EP 2 949 640 A1**
(43) Date of publication of application: **02.12.2015**
(21) Application number: 14742900.5
(22) Date of filing: 24.01.2014
(51) Int. Cl.: C07C 51/44, C07C 51/12, C07C 51/47, C07C 53/08, C07B 61/00

(54) **METHOD FOR MANUFACTURING CARBOXYLIC ACID**

(30) Priority: 25.01.2013 JP 2013011777
(71) Applicant: Daicel Corporation, Osaka 530-0011 (JP)
(72) Inventor: MIURA, Hiroyuki, Himeji-shi Hyogo 671-1283 (JP); SHIMIZU, Masahiko, Tokyo 108-8230 (JP); UENO, Takashi, Himeji-shi Hyogo 671-1281 (JP)
(74) Representative: Grünecker Patent- und Rechtsanwälte PartG mbB
(86) International application number: PCT/JP2014/051446
(87) International publication number: WO 2014/115826

(57) **Abstract**

A process and a production apparatus for producing a high-purity carboxylic acid, from which a metal-containing impurity and/or a carbonyl-group-containing impurity have been highly purified, are provided.

The process for producing a carboxylic acid according to the present invention comprises subjecting a carboxylic acid stream containing a metal-containing impurity to a first flash system 1 to give a vaporized fraction, distilling the vaporized fraction by a distillation system 5 to separate into a stream mainly containing a carboxylic acid and a fraction containing ahigh-volatile impurity (or lowerboilingpoint impurity), and feeding the separated stream mainly containing the carboxylic acid to a second flash system 10 or an adsorption system to form a purified carboxylic acid.

## Description

### TECHNICAL FIELD

The present invention relates to a process for producing a high-purity carboxylic acid (or a process for purifying a carboxylic acid) from which various impurities such as a metal-containing impurity and/or a carbonyl-group-containing impurity are removed. The metal-containing impurity may include a metal catalyst, a metal-containing catalyst stabilizer, and in addition, an impurity produced by corrosion of a metallic member or unit among various members or units (e.g., a reaction apparatus, a flash apparatus, a distillation apparatus, and a line for coupling these apparatuses), and other impurities. The carbonyl-group-containing impurity may include a carbonyl impurity, for example, a carboxylic acid other than an objective carboxylic acid, an aldehyde, an ester, and others.

### BACKGROUND ART

For industrial production, an aliphatic carboxylic acid (such as acetic acid) is practically obtained by allowing an alkanol having one less carbon atom than the carboxylic acid to react with carbon monoxide. The reaction system of the alkanol and carbon monoxide contains various components, (including a metal catalyst, a catalyst stabilizer or a co-catalyst (or a reaction accelerator), or a deactivated product thereof, and a reaction solvent, and a reaction product [e.g., a carboxylic acid, a reaction product or decomposed product (or polymer) of various components contained in the reaction system, and water generated by the reaction]. Thus in order to obtain a product carboxylic acid, it is necessary to remove these many impurities by a separation means such as distillation. Meanwhile, the components [in particular, e.g. , hydrogen iodide known as a catalyst stabilizer or a co-catalyst, a halide or a halide salt (such as a metal iodide), a halogen ion (such as an iodine ion), in addition to an inorganic acid, and an organic acid] contained in the reaction system (reaction liquid) are liable to corrode a reactor or a distillation system, or a peripheral equipment such as a line for conveying a reaction liquid or a separated liquid (or separated gas), particularly, a metallic equipment (metallic unit). Moreover, the corrosion of the reactor, the distillation system and the peripheral equipment produces a corroded metal which contaminates a carboxylic acid stream, so that the product carboxylic acid has a low quality or sometimes gets colored.

For these reasons, a method for removing impurities such as an iodide has been examined. For example, Japanese Patent Application Laid-Open Publication No. 6-40999 (JP-6-40999A, Patent Document 1) discloses a process for producing acetic acid, which comprises supplying methanol and carbon monoxide to a carbonylation zone that holds a liquid reaction composition consisting of a rhodium catalyst, methyl iodide, an iodide salt, water having a content up to about 10% by weight, methyl acetate having a concentration of at least 2% by weight, and acetic acid; introducing the liquid reaction composition into a flash zone; and recycling a liquid component from the flash zone to the reaction zone and collecting an acetic acid product from a vapor fraction of the flash zone by using simple distillation; wherein the vapor fraction from the flash zone is introduced into the distillation zone, a light end stream to be recycled is removed from the top of the distillation zone, and an acid product stream which has a water content of less than 1500 ppm and a propionic acid concentration of less than 500 ppm is withdrawn from the distillation zone. The document also discloses that an iodide as an impurity is removed from the acetic acidproduct by passing the acid product stream containing the acetic acid product through an ion exchange resin (anion exchange resin) bed and that the acid product stream is withdrawn at the base of the distillation zone or at a point 2 actual stages above the base of the distillation zone.
Unfortunately, reexaminations of the process according to the Patent Document 1 show that the concentration of hydrogen iodide in the product acetic acid cannot be reduced sufficiently (to not more than 12 ppm) under the condition of the water content of not more than 1500 ppm in the final product. Thus it is necessary to apply a larger load on the anion exchange resin, and this process is extremely unfavorable because of costs (furthermore, as unfavorable industrial process). Moreover, since the acid product stream is withdrawn from the base of the distillation zone, a less-volatile impurity (e.g., a metal-containing impurity) such as the rhodium catalyst is entrained in the acid product stream. Thus the quality of the product acetic acid is low, and the separability or recovery of the impurity cannot also be improved. Further, this process is costly disadvantageous because of an operational trouble due to clogging of a peripheral device (such as a line or a valve) or other factors, or because of an additional system (equipment) necessary for recovering the impurity or avoiding the operational trouble. Accordingly, the industrial or commercial operation of the above-mentioned process has a great risk.

International Publication No. WO02/062740 publication (Patent Document 2) discloses a continuous process for producing acetic acid comprising the following steps (a) to (d): (a) a step of reacting methanol with carbon monoxide; (b) a step of withdrawing a stream of a reaction medium from a reactor and vaporizing a portion of the withdrawn medium in a flashing step; (c) a step of distilling the flashed vapor utilizing two distillation columns to form a liquid acetic acid product stream; and (d) a step of removing iodides from the liquid acetic acid product stream such that the product stream has an iodide content of less than 10 ppb iodide by (i) contacting the liquid acetic acid product stream with an anionic ion exchange resin at a temperature of not lower than about 100°C followed by contacting the resultant stream with a silver or mercury exchanged ion exchange substrate or (ii) contacting the liquid acetic acid product stream with a silver or a mercury exchanged ion exchange substrate at a temperature of not lower than about 50°C. As described above, according to the Patent Document 2, the iodides are removed from the acetic acid product stream with the use of the anionic ion exchange resin and/or a guard bed. Unfortunately, according to the process of the Patent Document 2, it is difficult to sufficiently remove a less-volatile impurity such as a metal impurity or a sulfate. Moreover, since it is difficult to effectively remove a carbonyl impurity, which is a high-volatile impurity (or low boiling point impurity), such as an aldehyde, a carboxylic acid or an ester, the product acetic acid is low in a potassium permanganate test which is a standard of a product acetic acid and is of low quality. Therefore, in order to obtain acetic acid satisfying a standard of a product acetic acid according to the process of the Patent Document 2, it is necessary to install an incidental equipment for treating an acetic acid stream.

CN 1634842 A (Chinese Patent Publication, Patent Document 3) discloses a process for producing acetic acid having a formic acid impurity content of less than 50 ppm and a low iodine ion impurity content, comprising: passing acetic acid through an adsorption column 1 packed with a solid strong oxidizer insoluble in acetic acid to remove an impurity in the acetic acid, and further passing the resulting acetic acid through an adsorption column 2 packed with aluminum oxide and an adsorption column 3 packed with an activated carbon to further purify acetic acid. CN 1634843 A (Patent Document 4) discloses a process for obtaining acetic acid having a long potassium permanganate time, comprising: passing acetic acid through the adsorption column 1 to remove a reducing impurity, and further passing the resulting acetic acid through the adsorption columns 2 and 3 to further remove water and a saturated aldehyde in the acetic acid. Unfortunately, according to these processes, the iodine ion concentration cannot be reduced, as described in Examples of the Patent Documents 3 and 4.

Japanese PatentNo. 55-33428 (JP-55-33428B, Patent Document 5) relates to a process for purifying a carboxylic acid, which comprises feeding an alkylmonocarboxylic acid containing water and a halogen contaminant (including an alkyl halide and a halogenated hydrocarbon) to a first distillation zone (or first distillation column) and a second distillation zone (or second distillation column), and discloses that the driest acid product from the second zone is withdrawn as a bottom stream from the second distillation zone and that a product stream containing no trace of metal halide impurity can be withdrawn in vapor form from a point above the liquid level of the second column (second distillation column) bottom. Unfortunately, even in the case where the stream is withdrawn in vapor form from the point above the liquid level of the second distillation column bottom, the diameter of a withdrawing port or a withdrawing nozzle cannot be increased structurally. Thus the linear velocity of the withdrawn vapor is increased, so that impurities containing a metal component are entrained, resulting in a low quality of the product. In order to prevent the contamination due to the entrainment, a demister (a vapor-liquid separator for separating gas and mist, and an apparatus for collecting the separated mist as liquid and returning the liquid to the process) is required.

### RELATED ART DOCUMENTS

### PATENT DOCUMENTS

Patent Document 1: JP-6-40999A (Claims, paragraph No. [0043])
Patent Document 2: International Publication No. WO02/062740 publication (Claim 1)
Patent Document 3: CN 1634842 A (Claims, Examples)
Patent Document 4: CN 1634843 A (Claims, Examples)
Patent Document 5: JP-55-33428B (Column 9, lines 18 to 23)

### SUMMARY OF THE INVENTION

### PROBLEMS TO BE SOLVED BY THE INVENTION

It is therefore an object of the present invention to provide a process for producing a high-purity carboxylic acid from which a metal-containing impurity has been highly removed.

Another object of the present invention is to provide a process for producing a purified carboxylic acid, by which an impurity (such as a metal-containing impurity and/or a carbonyl impurity) can be removed efficiently by minimal steps.

It is still another object of the present invention to provide a process for producing a purified carboxylic acid, by which an impurity produced by corrosion of a metallic member or unit (e.g., a reaction system, a flash system, a distillation system, and/or a line for coupling these systems) can be removed effectively.

It is a further object of the present invention to provide a process for producing a high-purity carboxylic acid highly purified of a carbonyl impurity and a halide (such as an iodide), each of which causes lowering of a potassium permanganate test value and/or a potassium bichromate test value.

### MEANS TO SOLVE THE PROBLEMS

The inventors of the present invention made intensive studies to achieve the above objects and finally found that the purification of a carboxylic acid stream with combination of a first flash system, a distillation system, and a second flash system or an adsorption system in this order effectively removes not only a metal catalyst used for a reaction and/or an impurity (that is, a metal-containing impurity) produced by corrosion of a metallic unit (such as a reaction system, a flash system, or a distillation system) but also a carbonyl impurity having a boiling point higher or lower than a boiling point of an objective carboxylic acid, so that a purified carboxylic acid with a high purity can be obtained. The present invention was accomplished based on the above findings.

That is, the process for producing a carboxylic acid according to the present invention comprises: subjecting a carboxylic acid stream containing a metal-containing impurity to a first flash system to form (or give) a vaporized fraction; distilling the vaporized fraction by or in a distillation system to separate into a stream mainly containing a carboxylic acid and a volatile fraction containing a high-volatile impurity (or lower boiling point impurity); feeding the separated stream mainly containing the carboxylic acid to a second flash system or an adsorption system (for example, an adsorption system without the stream being oxidation-treated) to collect a purified carboxylic acid. The distillation system may comprise at least one distillation column or may comprise a plurality of distillation columns, for example, two distillation columns (a first distillation column and a second distillation column).

The carboxylic acid stream to be subjected to the first flash system may be a stream obtainable by allowing an alkanol to react with carbon monoxide in a reaction system in the presence of a metal catalyst [that is, e.g., a carboxylic acid stream which is obtainable by the reaction and contains the metal-containing impurity, or both metal-containing impurity and carbonyl-group-containing impurity (carbonyl impurity)]. The foregoing process applied to a stream containing a metal-containing impurity and/or a carbonyl impurity, and the carboxylic acid achieves efficient removal of the metal-containing impurity and the carbonyl impurity to produce a purified carboxylic acid.

Moreover, the carboxylic acid stream to be subjected to the first flash system may be a carboxylic acid (e.g., acetic acid) stream which is obtainable by allowing a C₁₋₄alkanol (e.g., methanol) to react with carbon monoxide in a reaction system in the presence of a rhodium catalyst, a metal halide (e.g., an alkali metal iodide) as a catalyst stabilizer, at least one co-catalyst selected from the group consisting of an alkyl halide (e.g., methyl iodide) and a hydrogen halide, and water and which contains a metal-containing impurity and a carbonyl impurity [for example, a carbonyl-group-containing impurity having a boiling point higher or lower than a boiling point of an objective carboxylic acid (e.g., a carboxylic acid, an aldehyde, a ketone, an ester, and an alkyl halide, each of which has carbon atoms more or less than carbon atoms in an objective carboxylic acid)].

At least one member (or unit) selected from the group consisting of the reaction system; the first flash system; and a stream feed line for coupling (or connecting) the reaction system with the first flash system may be made of a metal [made of an iron alloy (made of a stainless steel), made of a nickel alloy, made of metal zirconium, made of a zirconium alloy, made of a metal titanium, and made of a titanium alloy]. At least one member (or unit) selected from the group consisting of the reaction system; the first flash system; the distillation system; the stream feed line for coupling the reaction system with the first flash system; a stream feed line for coupling (or connecting) the first flash system with the distillation system; and a stream feed line for coupling (or connecting) the distillation system with the second flash system or the adsorption system may be made of the metal described above. Moreover, at least one member selected from the group consisting of the reaction system, the first and/or the second flash system, and the distillation system may be made of a nickel alloy, metal zirconium, or a zirconium alloy, and others. At least one member selected from the group consisting of the stream feed line for coupling the reaction system with the first flash system, the stream feed line for coupling the first flash system with the distillation system, and the stream feed line for coupling the distillation system with the second flash system or the adsorption system may be made of a stainless steel, and others.

The first flash system may comprise various flash distillation apparatuses, for example, a flash evaporation tank. The second flash system may comprise various flash distillation apparatuses, for example, a flash evaporation tank or a flash evaporator.

The stream containing the carboxylic acid from the distillation system may be subjected to the second flash system under a condition in which a temperature is 30 to 210°C and a pressure is 3 to 1,000 kPa for separating into a less-volatile (or low-volatile) impurity (or higher boiling point impurity (including a non-volatile impurity)) and the carboxylic acid. Use of the second flash system allows a port with a large diameter to be provided in a gaseous-phase region (or section) of the flash distillation apparatus. Thus the linear velocity of the vapor from the second flash system can easily be reduced, and the quality of the carboxylic acid can be improved while the entrainment of the metal-containing component can be prevented. Moreover, the process according to the present invention does not require an expensive demister having a complicated structure (a vapor-liquid separator, and an apparatus for returning a separated mist in the form of a liquid to a process). The flash distillation apparatus need not necessarily to be provided with a special unit. Only installation of a simple unit (e.g., a flow-controlling (or flow-regulating) unit) such as a baffle plate in the gaseous-phase region of the flash distillation apparatus can show the same effect as the case the demister is provided.

In the adsorption system, the stream mainly containing the carboxylic acid separated in the distillation system may be brought into contact with an adsorbent, without an oxidation treatment of the stream, for adsorbing a less-volatile impurity (or higher boiling point impurity (including a non-volatile impurity)) contained in the stream to the adsorbent to separate the carboxylic acid from the less-volatile impurity. The adsorption system usually contains no column packed with a solid strong oxidizer (such as potassium bromate), different from the Patent Documents 3 and 4. The treatment of the stream containing the carboxylic acid (carboxylic acid stream) with the solid strong oxidizer cannot effectively remove an iodine ion in the carboxylic acid stream probably due to formation of iodine (I₂) by oxidation of the iodine ion.

The metal-containing impurity comprises, for example, (i) at least one selected from the group consisting of a metal catalyst, a deactivated product thereof, a catalyst stabilizer, and a deactivated product thereof, and/or (ii) an impurity produced by corrosion of at least one metallic member or unit selected from the group consisting of the reaction system; the first flash system; and a stream feed line for coupling the reaction system with the first flash system.

The fraction containing the high-volatile impurity (or lower boiling point impurity) separated in the distillation system may be recycled to the reaction system. Moreover, the fraction containing the high-volatile impurity separated in the distillation system may be further subjected to an aldehyde separation system to remove an aldehyde contained in the high-volatile impurity for recycling the high-volatile impurity to the reaction system.

The stream containing the carboxylic acid collected from the second flash system or the adsorption system may be treated with an ion exchange resin. The treatment with the ion exchange resin may be carried out under a higher temperature.

In the production process, for example, the purified carboxylic acid having a potassium bichromate test value of not less than 140 minutes and a potassium permanganate test value of not less than 160 minutes may be collected.

The present invention also includes an apparatus for producing a carboxylic acid (or purified carboxylic acid), comprising: a first flash system for vaporizing (or flash-distilling) at least a carboxylic acid from a carboxylic acid stream containing a metal-containing impurity; a distillation system for distilling a vaporized fraction (a fraction containing the carboxylic acid) vaporized in the first flash system to form (or give) a stream mainly containing the carboxylic acid and a fraction containing a high-volatile impurity(or lower boiling point impurity); and a second flash system for purifying the stream mainly containing the carboxylic acid to give a purified carboxylic acid (a second flash system for vaporizing or flash-distilling the stream mainly containing the carboxylic acid) or an adsorption system (an adsorption system for absorption-treating the stream containing mainly the carboxylic acid).

### EFFECTS OF THE INVENTION

According to the present invention, because a carboxylic acid stream is purified with combination of a first flash system, a distillation system, and a second flash system or an adsorption system in this order, a metal-containing impurity can highly be removed from a product carboxylic acid, so that a high-purity carboxylic acid can be produced. Moreover, the present invention achieves efficient removal of an impurity (such as a metal-containing impurity and/or a carbonyl impurity) by minimal steps. The present invention achieves effective removal of an impurity (such as a metal-containing impurity) produced by corrosion of a metallic constituent member (or unit) (e.g., a reaction system, a flash system, a distillation system, and/or a line for coupling these systems). Further, the present invention allows high removal of the carbonyl impurity and production of a carboxylic acid having an improved potassium permanganate test value and/or potassium bichromate test value. Further, the treatment of the stream containing the carboxylic acid obtained from the second flash system or the adsorption system with an ion exchange resin can produce a high-purity carboxylic acid from which a halide such as an iodide (e.g., an alkyl iodide) has been highly removed.

### BRIEF DESCRIPTION OF DRAWINGS

[Fig. 1] Fig. 1 is a diagram for explaining a production process or a production apparatus of a carboxylic acid in accordance with an embodiment of the present invention.
[Fig. 2] Fig. 2 is a diagram for explaining a production process or a production apparatus of a carboxylic acid in accordance with another embodiment of the present invention.
[Fig. 3] Fig. 3 is a diagram for explaining a production process or a production apparatus of a carboxylic acid in accordance with still another embodiment of the present invention.

### DESCRIPTION OF EMBODIMENTS

Hereinafter, the present invention will be explained in detail with reference to the drawings if necessary.

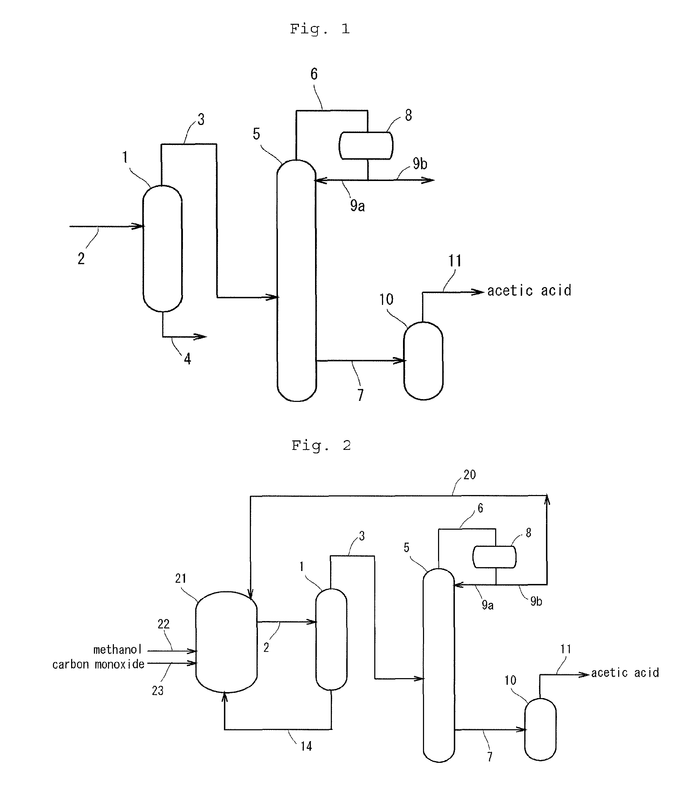

Fig. 1 is a diagram (a flow sheet, a schematic process drawing, or a schematic plant layout drawing) for explaining a production process (purification process) or a production apparatus of a purified carboxylic acid (such as acetic acid) in accordance with an embodiment of the present invention.

In the embodiment shown in Fig. 1, the apparatus for producing a purified carboxylic acid comprises : a first flash system (flash evaporation tank or distillation column made of a nickel alloy) 1 for vaporizing at least acetic acid from an acetic acid stream (a stream of acetic acid) containing an impurity; a distillation system (non-flash distillation system or distillation column) 5 for separating a fraction (a fraction containing acetic acid) vaporized in the flash distillation column 1 into a stream mainly containing acetic acid and a fraction containing a lower boiling point impurity (orhigh-volatile impurity); and a second flash system (flash evaporator) 10 for obtaining a purified acetic acid from the stream which is separated in the distillation column 5 and mainly contains acetic acid.

According to this apparatus, the acetic acid stream containing an impurity is subjected to the flash distillation column (or evaporation tank) 1 for vaporization, the resulting vaporized fraction is subjected to the distillation column 5 to give a separated stream mainly containing acetic acid, and the separated streamis subjectedtothe flash distiller 10 for vaporizing acetic acid, so that a highly purified acetic acid having less metal-containing impurity content can be collected.

More specifically, the acetic acid stream contains a metal-containing component such as a metal catalyst (e.g., a rhodium catalyst) and/or a metal-containing catalyst stabilizer (e.g., an alkali metal halide such as lithium iodide) used in the process of acetic acid production, and a metal-containing component produced by corrosion or other factors of a metallic member (or unit) such as a metallic reactor or a metallic line of acetic acid production process; the metal-containing component produced by corrosion may include, for example, a metal-containing impurity such as a metal halide, a metal salt of an inorganic acid (such as a metal sulfate), or a metal salt of an organic acid (such as a metal carboxylate). The metal element contained in the metal-containing component may include various metal elements such as a transition metal element (such as iron or nickel) and a typical metal element (such as an alkali metal, an alkaline earth metal, or aluminum). The acetic acid stream containing such a metal-containing impurity is fed to the flash distillation column 1 via a feed line 2. The feed line 2 may be made of a corrosion-resistant metal, for example, a nickel alloy, zirconium, and others. The flash distillation column 1 separates the stream into a volatile component and a less-volatile component by vaporization. The volatile component [orahigh-volatilecomponent (lower boiling point component)] may include, for example, acetic acid as the main product, as well as water, various components used in the production process (for example, raw material methanol, and methyl iodide as a co-catalyst), a reaction by-product (or polymer or decomposed product) of components used in the production process, e.g., formic acid, propionic acid, and crotonic acid, and methyl esters thereof; an alkyl iodide such as pentyl iodide or hexyl iodide; and an aldehyde such as acetaldehyde or crotonaldehyde). The less-volatile component [or higher boiling point component (including a non-volatile component)] may include the metal-containing impurity (in particular, a metal catalyst such as a rhodium catalyst, a metal-containing catalyst stabilizer such as lithium iodide). The less-volatile component is withdrawn (or discharged) from the flash system via a withdrawing (or discharge) line 4, and the stream withdrawn from the withdrawing line 4 can suitably be recycled to an acetic acid production step (not shown). The stream from the withdrawing line 4 need not necessarily be recycled to the acetic acid production step. Since the stream usually contains a component useful for producing acetic acid, for example, the metal-containing impurity and a remaining component that does not vaporize or evaporate (e.g., a catalyst, a co-catalyst, a catalyst stabilizer, methyl iodide, methyl acetate, hydrogen iodide, water, and acetic acid), the stream is advantageously recycled to the production step. The stream (fraction) containing acetic acid vaporized in the flash distillation column 1 is withdrawn (or distilled off) from the flash system via a withdrawing line or feed line 3 and fed to the distillation column (non-flash distillation column) 5. Each of the withdrawing line or feed line 3 and the withdrawing line 4 is usually made of a highly corrosion-resistant metal, for example, a nickel alloy, zirconium, and/or a stainless steel.

The distillation column 5 is made of a corrosion-resistant metal (for example, a nickel alloy and zirconium). In the distillation column 5, the fed stream containing acetic acid is distilled for separating into a fraction containing a lower boiling point impurity [for example, water, hydrogen iodide, methyl iodide, and a carbonyl impurity having a boiling point lower than that of acetic acid (e.g., formic acid, an ester such as methyl acetate, and an aldehyde such as acetaldehyde)] and a stream containing a further purified acetic acid which is collected. In more detail, the stream containing the purified acetic acid is withdrawn from the bottom of the distillation column 5 via a line 7, and the fraction containing the lower boiling point impurity is withdrawn from the top of the distillation column 5 via a withdrawing line 6. Each of the withdrawing line 6 and the line 7 is made of a corrosion-resistant metal, for example, a high-class material (such as a nickel alloy) and/or a stainless steel.

The fraction from the top of the distillation column 5 may be fed to a heat exchanger (condenser) 8 via the withdrawing line 6 and may be condensed (or liquefied) by the condenser 8. A portion of the fraction (condensate) condensed by the condenser 8 may be returned and refluxed to the upper part of the distillation column 5 via a reflux line 9a, and the remainder of the condensate is withdrawn via a withdrawing line 9b. The reflux of the condensate is not necessarily needed. All of the condensate may be withdrawn (in other words, the reflux ratio may be zero) . The reflux (returning) of the condensate to the upper part of the distillation column 5 allows efficient countercurrent contact of the condensate with a gaseous component in the distillation column 5, which achieves a high rectification effect. Thus the lower boiling point impurity and acetic acid can be separated effectively from each other. The heat exchanger (condenser) 8 may be made of a corrosion-resistant metal, for example, a high-class material (such as a nickel alloy) and/or a stainless steel. Each of the reflux line 9a and the withdrawing line 9b may be made of a corrosion-resistant metal, for example, a stainless steel.

The condensate withdrawn via the condensate withdrawing line 9b can suitably be recycled to the acetic acid production step (not shown). If necessary, before the condensate is recycled to the production step, a component (e.g., acetaldehyde) that hinders the reaction in the production step may be removed. In a case where the condensate from which acetaldehyde has been removed is returned (recycled) to the acetic acid production step [for example, the reaction system (e.g., a carbonylation reaction system)], the quantity of by-products (e.g., propionic acid, a substance harmful to potassium permanganate test, and an alkyl iodide) can be reduced in the reaction system. This results in easy purification of acetic acid in the succeeding distillation system.

Moreover, the condenser 8 is not necessarily needed. The gaseous fraction from the distillation column and/or the condenser may be discharged out of the system or may be collected. The condensate in the condenser 8 may further be fed to a stainless-steel decanter (not shown) to form (or separate) an upper phase (aqueous phase) and a lower phase (organic phase). Moreover, a portion or all of the lower phase and/or the upper phase formed in the decanter may be refluxed to the distillation column 5, and the remainder maybe recycled to the carboxylic acid production process.

A stream collected from the lower part of the distillation column 5 is rich in acetic acid and contains other impurities, in particular, an entrained less-volatile component (or higher boiling point component). The less-volatile component may include, for example, a higher boiling point component (e.g., a metal-containing impurity such as iron, a metal sulfate, or a metal halide) produced by corrosion of various metallic units, for example, a corrosion-resistant metallic (e.g., a nickel-alloy) distillation column 5, a metallic (e.g., a stainless-steel) line (for example, withdrawing lines 3 and 6, a reflux line 9a, and a feed line 7), and a metallic (e.g., a stainless-steel) peripheral unit (for example, a condenser 8, a decanter, and a reboiler (not shown)). Thus according to the present invention, the stream containing acetic acid collected from the lower part of the distillation column 5 is further fed to a flash evaporator 10 via the line (or feed line) 7 to remove a higher boiling point component. That is, due to a flash phenomenon in the flash evaporator 10, a component containing acetic acid (lower boiling point component) vaporizes from the stream containing acetic acid fed to the flash evaporator 10, and a higher boiling point component settles in the bottom of the flash evaporator 10. The component containing acetic acid (lower boiling point component) vaporized in the flash evaporator 10 is withdrawn from the head (or top) of the flash evaporator 10 via a line (withdrawing line) 11 and collected as a product acetic acid. Each of the flash evaporator 10 and the line (withdrawing line) 11 is usually made of a corrosion-resistant metal such as a high-class material (such as a nickel alloy) and/or a stainless steel. The product acetic acid is highly purified because of removal of a higher boiling point component (less-volatile component) such as a metal-containing impurity and untinged or colorless, and has a potassium permanganate test, which is a product standard, of not less than 120 minutes (in particular, not less than 160 minutes), and a high purity. Moreover, use of the flash distiller 10 can inhibit the entrainment of the lower boiling point component while decreasing the linear velocity of the lower boiling point component. Thus the quality of the purified carboxylic acid can be improved without use of an expensive demister having a complicated structure.

If necessary, the acetic acid stream from the head of the flash evaporator 10 may further be treated with an ion exchange resin or the like (not shown) so that the removal rate of the higher boiling point impurity [or less-volatile impurity, for example, an alkyl iodide such as hexyl iodide (e.g., an alkyl iodide having a lower volatility or a higher boiling point than those of acetic acid)] can further be increased.

The production process (purification process) of the present invention can be applied to a production system for producing a purified carboxylic acid from a carboxylic acid stream containing a metal-containing impurity and is useful for separation of a reaction liquid (crude reaction liquid) containing, in addition to the metal-containing impurity, various components produced by a carbonylation reaction of an alkanol or a derivative thereof (e.g., anester) or other reactions. As used herein, an alkanol derivative (a carbonylatable alkanol derivative) as well as an alkanol may be referred to as "alkanol" simply.

Fig. 2 is a diagram for explaining a production process or a production apparatus of a purified carboxylic acid (e.g., acetic acid) in accordance with another embodiment of the present invention. The apparatus shown in Fig. 2 comprises a reaction system (reactor) 21 for forming or producing a carboxylic acid (e.g., acetic acid) by allowing an alkanol to react with carbon monoxide, in addition to the embodiment of Fig. 1. A reaction liquid from the reactor 21 is fed to a flash distillation column (or evaporation tank) 1 via a feed line 2. Each of the reaction system (reactor) 21 and the feed line 2 is usually made of a corrosion-resistant metal such as a nickel alloy or zirconium. In the embodiment of Fig. 2, the flash distillation column 1 and subsequent steps or units are the same as the embodiment of Fig. 1, and a purified acetic acid is collected from the head of a flash evaporator 10. That is, the apparatus shown in Fig. 2 comprises the flash distillation column 1, the distillation column 5, the flash evaporator 10, and in addition, the reactor 21 for feeding, to the flash distillation column 1, a carboxylic acid (e.g., acetic acid) stream containing ametal-containing impurity (for example, a metal catalyst such as a rhodium catalyst, and a metal halide such as lithium iodide as a catalyst stabilizer) and a carbonyl impurity having a boiling point higher or lower than that of an objective carboxylic acid.

In the embodiment of Fig. 2, a catalyst system (e.g., a rhodium catalyst, lithium iodide as a catalyst stabilizer, and methyl iodide as a co-catalyst) is contained in the reactor 21, and methanol and carbon monoxide, which are raw materials of acetic acid, are fed to the reactor 21 via feed lines 22 and 23, respectively. Inside the reactor 21, methanol is allowed to react with carbon monoxide in the presence of the catalyst system to produce acetic acid, and thus a reaction liquid (crude reaction liquid) containing acetic acid is produced. The reaction liquid contains acetic acid, and in addition, water, other carboxylic acids [e.g., a lower or higher carboxylic acid than acetic acid (a saturated or unsaturated carboxylic acid) such as formic acid, propionic acid, or crotonic acid], esters of these other carboxylic acids or acetic acid (e.g., an ester of a carboxylic acid with raw material methanol), an aldehyde (e.g., a saturated or unsaturated aldehyde such as acetaldehyde, crotonaldehyde, or 2-ethylcrotonaldehyde), raw material methanol, the catalyst system, an alkyl halide [e.g., an alkyl iodide, for example, a C₂₋₁₅alkyl iodide (preferably a C₂₋₁₂alkyl iodide) such as hexyl iodide or decyl iodide], and others. Accordingly, the reaction liquid is fed to the flash distillation column (evaporation tank) 1 via a flow (or feed) line, and a less-volatile component such as the rhodium catalyst or lithium iodide is separated from the bottomof the flashdistillation column 1. Since the stream from the bottom of the flash distillation column 1 contains a useful component for the reaction system, such as the catalyst system (e.g., the rhodium catalyst and lithium iodide), the stream is recycled to the lower part (orbottom) of the reactor 1 via a recycle line 14. The recycle line 14 is made of a corrosion-resistant metal such as a nickel alloy.

To the reactor 21, if necessary, hydrogen may be fed in order to increase the catalytic activity. Hydrogen may be fed together with carbon monoxide via a feed line 23 or may be fed separately via another feed line (not shown).

Moreover, in the embodiment of Fig. 2, a fraction withdrawn from the top of the evaporation column 5 is, in the same manner as the embodiment of Fig. 1, fed to a condenser 8 via a withdrawing line 6 and condensed by the condenser 8, and a portion of the resulting condensate is returned to the upper part of the distillation column 5 via a reflux line 9a. Moreover, since the condensate contains a useful component for the reaction system (e.g., hydrogen iodide and methyl iodide), the remainder of the condensate is recycled to the upper part (including a top such as a head) of the reactor 21 via a withdrawing line 9b and a recycle line 20. Prior to the recycling of the condensate to the reactor 21, if necessary, a component (e.g., acetaldehyde) that hinders the reaction may be removed from the condensate. Each of the withdrawing line 6 and the condenser 8 may be made of a corrosion-resistant metal such as a nickel alloy; each of the reflux line 9a, the withdrawing line 9b, and the recycle line 20 may also be made of a corrosion-resistant metal such as a stainless steel.

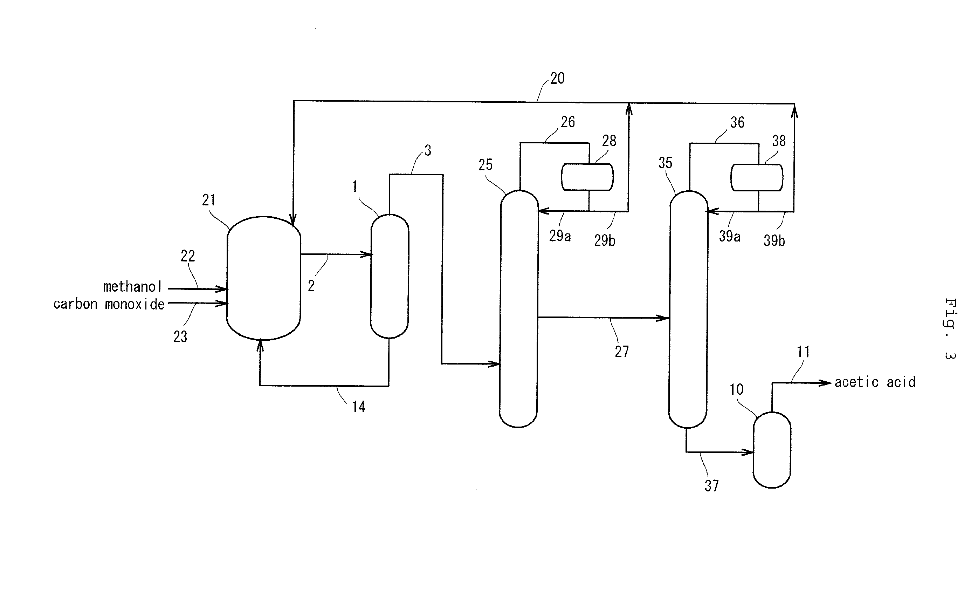

Fig. 3 is a diagram for explaining a production process or a production apparatus of a purified carboxylic acid (e.g., acetic acid) in accordance with still another embodiment of the present invention. The apparatus shown in Fig. 3 has two non-flash distillation columns, that is, a first distillation system (first distillation column (or column for separating a lower boiling point component)) 25 and a second distillation system (second distillation column (or dehydration column)) 35, in place of the distillation column 5 in the embodiment of Fig. 2.

In more detail, the apparatus shown in Fig. 3 comprises: a reaction system (reactor) 21 for forming or producing acetic acid by allowing methanol to react with carbon monoxide; a flash distillation column 1 for separating a reaction liquid (crude reaction liquid) fed from the reactor 21 into a fraction (stream) containing acetic acid and a less-volatile (non-volatile) component by vaporization; a first distillation column 25 for separating the stream containing acetic acid fed from the flash distillation column 1 into a lower boiling point impurity [such as hydrogen iodide, methyl iodide, or a lower boiling point carbonyl impurity(e.g., methylacetate and acetaldehyde)] and a stream mainly containing acetic acid (and water); a second distillation column 35 for separating the stream containing acetic acid fed from the first distillation column 25 into a stream containing water and a stream containing acetic acid; and a flash evaporator 10 for separating the stream containing acetic acid fed from the second distillation column 35 into a purified acetic acid and a non-volatile component (such as a metal-containing impurity) by vaporization. Each of the first distillation column 25, the second distillation column 35, and the flash evaporator 10 is usually made of a corrosion-resistant metal, for example, a high-class material (such as a nickel alloy) and/or a stainless steel.

In the embodiment of Fig. 3, in the same manner as in the embodiment of Fig. 2, the reaction liquid from the reactor 21 is fed to the flash distillation column 1 via a line 2, a vaporized stream containing acetic acid vaporized in the flash distillation column 1 is withdrawn from the top of the column via a withdrawing line 3, and a higher boiling point component is recycled to the reactor 21 from the bottom of the flash distillation column via a recycle line 14. The higher boiling point component includes a metal-containing impurity, for example, a metal-containing catalyst component (such as a rhodium catalyst or lithium iodide) and a metal-containing component produced by corrosion of various metallic units (or members) constituting the apparatus (e.g., a nickel-alloy reactor 21 and a recycle line 14) according to the present invention. Each of the line 2, the withdrawing line 3, and the recycle line 14 may be made of, for example, a corrosion-resistant metal such as a high-class material (e.g., a nickel alloy) and/or a stainless steel.

In the embodiment of Fig. 3, the stream containing acetic acid withdrawn from the flash distillation column (evaporation tank) 1 made of a nickel alloy or the like is firstly fed to a first distillation column (column for separating a lower boiling point component) 25 made of a nickel alloy or the like for withdrawing a lower boiling point impurity [e.g., a halide such as hydrogen iodide or methyl iodide, and a lower boiling point carbonyl impurity (e.g., methyl acetate and an aldehyde such as acetaldehyde)], a portion of water, a portion of acetic acid, and other components from the top of the column via a withdrawing line 26. A stream containing acetic acid (and water) is withdrawn from the middle part of the first distillation column 25 by side cut via a line 27 made of a nickel alloy or the like. The stream containing acetic acid from the first distillation column 25 by side cut is fed to the middle part of a second distillation column 35 made of a nickel alloy or the like for distilling and separating the stream into a fraction containing water and a stream containing acetic acid by utilizing difference between acetic acid and water in boiling point. The fraction containing water is discharged or withdrawn from the top of the column via a withdrawing line 36 made of a nickel alloy or the like, and the stream containing acetic acid is withdrawn from the bottom of the column via a bottom line 37 made of a high-class material (such as a nickel alloy) and/or a stainless steel, or the like. The stream containing acetic acid from the bottom of the second distillation column 35 is fed to a flash evaporator 10 made of a high-class material (such as a nickel alloy) and/or a stainless steel or the like via the bottom (or feed) line 37. In the same manner as the embodiments of Fig. 1 and Fig. 2, acetic acid is separated from a higher boilingpoint component suchas ametal-containing impurity [for example, a metal-containing component produced by corrosion of various metallic units (or members), such as units such as the reactor 21, the flash distillation column 1, the first distillation column 25, the second distillation column 35, and condensers 28 and 38, and lines for coupling these units each other], and the purified acetic acid is withdrawn from the head of the evaporator 10 via a line 11. Post-treatment or other treatments for the acetic acid stream withdrawn from the head of the flash evaporator 10 may be carried out in the same manner as the embodiment of Fig. 1.

The fraction withdrawn from the top of the first distillation column 25 via the withdrawing line 26 made of a nickel alloy or the like, and the fraction withdrawn from the top of the second distillation column 35 via the withdrawing line 36 made of a nickel alloy or the like are fed to the condensers 28 and 38, each made of a nickel alloy or the like, and condensed therein, respectively. In the same manner as in the case of the distiller 5 in Fig. 2, a portion of the condensate in the condenser 28 is refluxed to the upper part (e.g., top) of the first distillation column 25 via a reflux line 29a made of a stainless steel, and the remainder is withdrawn via a withdrawing line 2 9b made of a stainless steel; and a portion of the condensate in the condenser 38 is refluxed to the upper part (e.g., top) of the second distillation column 35 via a reflux line 39a made of a stainless steel, and the remainder is withdrawn via a withdrawing line 39b made of a stainless steel. The condensates withdrawn via the withdrawing lines 29b and 39b are introduced to a recycle line 20 made of a stainless steel and recycled to the upper part (e.g., head or top) of the reactor 21 via the recycle line 20.

In the embodiment of Fig. 3, in the same manner as the embodiments of Fig. 1 and Fig. 2, prior to the recycling of each condensate to the reaction system (reactor 21), if necessary, a component (e.g., acetaldehyde) that hinders the reaction may be removed from the condensate. In the embodiment of Fig. 3, in a case where acetaldehyde is removed from the condensate before the condensate is recycled to the reactor 21, the quantity of by-products (e.g., propionic acid, a substance harmful to potassium permanganate test, and an alkyl iodide) is reduced in the reactor 21. This can result in an extremely low concentration of propionic acid (for example, a concentration that satisfies the standard of acetic acid product) in the stream containing acetic acid to be fed from the first distillation column 25 to the second distillation column 35. Thus it is not necessary to take additional treatment for removing propionic acid in the second distillation column 35 and subsequent steps.

Fig. 3 illustrates that the distillation system (non-flash distillation system) comprises the column 25 for separating a lower boiling point component and the succeeding dehydration column 35. The order of the columns may be reversed.

Hereinafter, each member (or unit) in the production process (or apparatus) of the present invention will be explained in detail.

### [Reaction system or reaction step]

In the reaction system, an alkanol (or a derivative thereof) is allowed to react with carbon monoxide in the presence of a metal catalyst for carbonylation of the alkanol or the derivative thereof with carbon monoxide to produce a corresponding carboxylic acid.

### (Alkanol or derivative thereof)

The alkanol for the carbonylation reaction may include an alkanol having "n" carbon atom(s), for example, a C₁₋₁₀alkanol such as methanol, ethanol, propanol, isopropanol, butanol, pentanol, or hexanol. The number of carbon atoms, "n", is preferably about 1 to 6, more preferably about 1 to 4, and particularly about 1 to 3.

The derivative of the alkanol may include a reactive derivative such as an ester, an ether, or a halide. Among these derivatives, the ester may include an ester of a produced carboxylic acid with a raw material alcohol, for example, a C₁₋₆alkyl C₂₋₆carboxylate such as methyl acetate or ethyl propionate. As the ether, there may be mentioned an ether corresponding to the raw material alcohol, for example, a diC₁₋₆alkyl ether such as a methyl ether, an ethyl ether, a propyl ether, an isopropyl ether, or a butyl ether. As the halide, there may be used a halide corresponding to the alcohol (e.g., an alkyl halide such as an alkyl iodide), such as methyl iodide.

The alkanols or the derivatives thereof maybe used alone or in combination.

In a preferred liquid-phase reaction system, the alkanol having "n" carbonatom(s), preferably a C₁₋₄alkanol or a derivative thereof (for example, methanol, methyl acetate, and dimethyl ether) maybeusedas a liquid reactant to give a carboxylic acid having "n+1" carbon atoms or a derivative thereof (suchas a carboxylic acid anhydride) . In particular, in a preferred reaction system, for example, at least one member selected from the group consisting of methanol, methyl acetate, and dimethyl ether (in particular, methanol) is allowed to react with carbon monoxide in a liquid-phase reaction system in the presence of a carbonylation catalyst system to form acetic acid or a derivative thereof.

The alkanol or the derivative thereof as a fresh raw material may be fed to the reaction system directly or indirectly, or the alkanol or the derivative thereof withdrawn from the distillation step may be recycled to the reaction system.

Moreover, the reaction system may contain a carboxylic acid ester (in particular, an ester of a carboxylic acid with an alkanol, such as methyl acetate) at a proportion of about 0.1 to 35% by weight, preferably about 0.3 to 20% by weight, and more preferably about 0.5 to 10% by weight (e.g. , about 0.5 to 6% by weight) in the whole liquid-phase reaction system. In the reaction liquid, the carboxylic acid ester usually exists in equilibrium with the raw material alkanol and the product carboxylic acid.

### (Catalyst)

The reaction is carried out in the presence of a carbonylation catalyst. As the carbonylation catalyst, there may be usually employed a catalyst having a high boiling point, e.g., a metal catalyst. As the metal catalyst, there may be exemplified a transition metal catalyst, in particular a metal catalyst containing a group 8 metal of the Periodic Table, for example, a cobalt catalyst, a rhodium catalyst, an iridium catalyst, or others. The catalyst may be a simple metal, or may be used in the form of a metal oxide (including a complex metal oxide), an metal hydroxide, ametalhalide (e.g., a chloride, a bromide, an iodide), a metal carboxylate (e.g., an acetate), a metal salt of an inorganic acid (e.g., a sulfate, a nitrate, a phosphate), a metal complex or others. Further, the catalyst may be a supported catalyst in which a catalyst component is supported on a carrier or a support. Such a metal catalyst may be used singly or in combination.

The preferred metal catalyst includes a rhodium catalyst and an iridium catalyst (in particular a rhodium catalyst). Further, it is preferred to use the metal catalyst in the form dissolvable in a reaction liquid. Incidentally, since rhodium usually exists as a complex in the reaction liquid, in a case where a rhodium catalyst is used, the catalyst is not particularly limited and may be used in various forms as far as the catalyst can change into a complex in the reaction liquid. As such a rhodium catalyst, a rhodium halide (such as bromide or iodide) is particularly preferred. Moreover, the catalyst may be stabilized in the reaction liquid by adding a salt of a halide (e.g., a salt of an iodide) and/or water thereto.

The concentration of the catalyst is, for example, about 10 to 5,000 ppm, preferably about 200 to 3, 000 ppm, more preferably about 300 to 2,000 ppm, and particularly about 500 to 1,500 ppm on the basis of weight in the whole liquid phase system.

The carbonylation catalyst may usually be employed in combination with a catalyst stabilizer (or reaction accelerator) and/or a co-catalyst to form a catalyst system.

The catalyst stabilizer may include an alkali metal halide [for example, an alkali metal iodide (such as lithium iodide, potassium iodide, or sodium iodide), an alkali metal bromide (such as lithium bromide, potassium bromide, or sodium bromide), and a chloride and a fluoride, each corresponding to the iodide or bromide], a quaternary ammonium salt or quaternary phosphonium salt of an alkali metal (such as lithium, potassium, or sodium), a compound capable of forming an iodide salt in the reaction system, and others. These catalyst stabilizers may be used alone or in combination.

The catalyst stabilizer has an effect in preventing destabilization of the metal catalyst or reduction of the reaction rate. Among these components, from the viewpoint of the solubility or the stability of the catalyst (e.g., a rhodium catalyst), it is preferred to use an alkali metal iodide, particularly lithium iodide.

Moreover, the proportion of the catalyst stabilizer (such as lithium iodide) may for example be about 0.1 to 40% by weight, preferably about 0.5 to 35% by weight, and more preferably about 1 to 30% by weight in the whole liquid-phase reaction system. In particular, the catalyst stabilizer is practically contained in the liquid-phase reaction system in a proportion so that the concentration of an ionic halide (such as an iodide) can be maintained at, e.g., about 1 to 30% by weight and preferably about 2 to 20% by weight in the liquid-phase reaction system.

As the co-catalyst, there may be used a hydrogen halide (such as hydrogen iodide or hydrogen bromide), an alkyl halide [for example, an alkyl halide corresponding to the raw material alkanol (a C₁₋₁₀alkyl halide, preferably a C₁₋₄alkyl halide), e.g., a C₁₋₁₀alkyl iodine (such as a C₁₋₄alkyl iodide) such as methyl iodide, ethyl iodide, or propyl iodide, and a bromide (such as methyl bromide or propyl bromide) or a chloride (such as methyl chloride), each corresponding to the alkyl iodide], and others. The co-catalysts may be used alone or in combination. Among these co-catalysts, an alkyl iodide (such as a C₁₋₃alkyl iodide) such as methyl iodide is preferred. The alkyl iodide may be used in combination with hydrogen iodide.

The co-catalyst content of the whole liquid-phase reaction system may be, for example, about 0.1 to 40% by weight, preferably about 0.5 to 30% by weight, and more preferably about 1 to 25% by weight (e.g., about 3 to 20% by weight). More specifically, in the process for producing a carboxylic acid by the carbonylation reaction of the alkanol, the alkyl halide (e.g., methyl iodide) content of the whole liquid-phase system may be, for example, about 1 to 25% by weight, preferably about 2 to 20% by weight, and more preferably about 6 to 16% by weight. A higher concentration of the alkyl halide tends to accelerate the reaction.

The preferred catalyst system may include a catalyst system containing a rhodium catalyst, a metal halide (such as an alkali metal iodide) as a catalyst stabilizer, and at least one co-catalyst (particularly, such as methyl iodide) selected from the group consisting of an alkyl halide (suchas a C₁₋₃alkyl iodide) and a hydrogen halide (such as hydrogen iodide).

In the preferred reaction system, the C₁₋₄alkanol (such as methanol) may be allowed to react with carbon monoxide in the reaction system in the presence of the foregoing catalyst system and water to give a carboxylic acid stream (a stream of a carboxylic acid) containing a metal-containing impurity.

### (Carbon monoxide)

The carbon monoxide to be fed to the reaction system may be used as a pure gas or may be used as a gas diluted with an inactive gas (e.g., nitrogen, helium, and carbon dioxide). Moreover, since an exhausted gas containing carbon monoxide can be separated from the succeeding step(s) [e.g., a flash system, a distillation step (distillation column), and a condenser], carbon monoxide may be fed to the reaction system by recycling the exhausted gas containing carbon monoxide to the reaction system.

The method for feeding carbon monoxide is not particularly limited to a specific one. For example, carbon monoxide may be fed to the gaseous phase in the reaction system (reactor). Preferably, carbon monoxide is usually fed to the liquid phase in the reaction system (reactor). For example, carbon monoxide may be fed to the liquid phase in the reactor by bubbling or may be fed from the lower part (or bottom) of the reactor by sparging.

The carbon monoxide partial pressure in the reaction system may be, for example, about 0.8 to 3 MPa (e.g., 0.9 to 2 MPa), preferably about 1 to 2.5 MPa (e.g., about 1.15 to 2.5 MPa), and more preferably about 1.15 to 2 MPa (e.g., 1.18 to 2 MPa) as an absolute pressure.

### (Others)

The concentration of water in the reaction system is not particularly limited to a specific one and may be low. The water concentration of the reaction system may be, for example, about 0.01 to 10% by weight, preferably about 0.1 to 8% by weight, and more preferably about 0.5 to 7% by weight (particularly, about 1 to 5% by weight) in the whole liquid phase of the reaction system.

In the carbonylation reaction, hydrogen is usually formed (or generated) by a shift reaction between carbon monoxide and water. Hydrogen may be fed to the reaction system. The hydrogen may be fed as a mixed gas with carbon monoxide as a rawmaterial to the reaction system. Moreover, gaseous component (s) (including hydrogen, carbon monoxide , and others) may be collected from the succeeding distillation step(s) (distillation column(s)) or condenser(s). The hydrogen may be fed to the reaction system by recycling the gaseous component (s), if necessary after suitably purifying the gaseous component(s).

The hydrogen partial pressure in the reaction system may for example be about 0.5 to 200 kPa, preferably about 1 to 120 kPa, and more preferably about 5 to 100 kPa in terms of absolute pressure.

The carbon monoxide partial pressure or hydrogen partial pressure in the reaction system may be adjusted, for example, by suitably adjusting the amount of the carbon monoxide and hydrogen fed and/or recycled to the reaction system, the amount of raw substances (e.g., methanol) fed to the reaction system, the reaction temperature, the reaction pressure, and others.

In the carbonylation reaction, the reaction temperature may be, for example, about 100 to 250°C, preferably about 150 to 220°C, and more preferably about 170 to 200°C. Moreover, the reaction pressure may be about 1 to 5 MPa, preferably about 1. 5 to 4 MPa, and more preferably about 2 to 3.5 MPa in terms of gauge pressure.

The reaction may be carried out in the presence or absence of a solvent. The reaction solvent is not limited to a specific one as far as the reactivity, the separation or purification efficiency does not decrease, and a variety of solvents may be used. In usual cases, a carboxylic acid (e.g., acetic acid) as a product may be practically utilized as a solvent.

In the foregoing carbonylation reaction system, the production of a carboxylic acid (e.g., acetic acid) having "n+1" carbon atoms corresponding to the alkanol (e.g., methanol) having "n" carbon atom(s) is accompanied by the formation of by-products, for example, a carbonyl impurity (such as a carboxylic acid ester, an aldehyde, or a ketone) and a halide (such as an alkyl iodide) in addition to by-product carboxylic acids other than the objective carboxylic acid. More specifically, the by-products may include an ester (e.g., methyl acetate) of the produced carboxylic acid with the raw material alkanol; and water generated by esterification; analdehyde (e.g., acetaldehyde) having "n+1" carbon atoms, corresponding to the alkanol; and a carboxylic acid (e.g., propionic acid) having "n+2" carbon atoms; and a reaction product, polymer and decomposed product of various components contained in the reaction system [for example, other carboxylic acids (e.g., formic acid and crotonic acid), other aldehydes (e.g., crotonaldehyde), other esters (e.g., methyl propionate and methyl crotonate), and an iodide (e.g., a C₂₋₁₅alkyl iodide (particularly, a C₂₋₁₂alkyl iodide) such as pentyl iodide, hexyl iodide, or decyl iodide)].

In the reaction system, generation of aldehydes may be suppressed or inhibited by removing the aldehyde (e.g., acetaldehyde) in the recycling stream from the succeeding step(s) (e.g., distillation system), or by modifying the reaction conditions, for example, reducing the proportion of the co-catalyst such as an alkyl iodide and/or the hydrogen partial pressure. Moreover, the generation of hydrogen in the reaction system may be suppressed or inhibited by adjusting the concentration of water.

The species of the reaction apparatus (or reactor) is not particularly limited to a specific one. The reaction apparatus to be used may include a conventional carbonylation reactor, for example, astirred-tank reactor (e.g., a continuous, batch, or semi-batch stirred-tank reactor), a tubular reactor, a column reactor, a fixed-bed reactor, and a fluidized-bed reactor.

Moreover, a member or unit made of a conventional material may be used as the reaction system (reaction apparatus or reactor) and the peripheral unit of the reaction system (for example, various lines communicating to the reaction system, e.g., a line for coupling the reaction system with any other unit, such as a feed line or a stream-withdrawing or stream-discharging line). The conventional material may include, for example, a glass, a metal [for example, a metal or a metal alloy, e.g., (i) an iron alloy such as a stainless steel (for example, a martensitic stainless steel such as Fe-Cr series (e.g., SUS410) or Fe-Cr-Ni series precipitation hardening (e.g., SUS630); a ferritic stainless steel such as Fe-Cr series (e.g., SUS430); an austenitic stainless steel such as Fe-Cr-Niseries (e.g., SUS304, SUS316L) ; a semi-austenitic stainless steel such as Fe-Cr-Ni series precipitation hardening (e.g., SUS631); and an austenitic-ferritic stainless steel such as Fe-Cr-Ni series (e.g., SUS329J1)); and (ii) a metal (such as nickel, zirconium, titanium, chromium, tantalum, molybdenum, manganese, cobalt, or tungsten) or an alloy containing a plurality of these metals (for example, pure nickel or a nickel alloy, such as Ni200, Ni201, MONEL (registered trademark) 400, or MONEL (registered trademark) K-500)], and a ceramic. Among these materials, the preferred material for the reaction system and/or the peripheral unit thereof includes a metal or metal alloy having a corrosion resistance (e.g., a corrosion resistance to an acid, and a corrosion resistance to an oxidizing atmosphere), for example, a corrosion-resistant stainless steel (e.g., NAS254N, NAS354N, NAS185N and NAS155N, which are manufactured by Nippon Yakin Kogyo Co., Ltd.; a nickel-containing stainless steel such as Carpenter 20cb3, and a Fe-Cr-Ni series austenitic stainless steel (e.g., SUS304, SUS316L)), a high-class material, for example, a nickel alloy (e.g., a nickel alloy containing nickel and at least one member selected from the group consisting of chromium, molybdenum, manganese, iron, cobalt, tantalum, and tungsten), metal zirconium (Zr), a zirconium alloy, metal titanium (Ti), and a titanium alloy.

Concrete examples of the nickel alloy includes, for example, a Ni-Mo series alloy [for example, a HASTELLOY (registered trademark) B series (e.g., HASTELLOY B-2 (registered trademark)) manufactured by Haynes International]; a Ni-Cr-Mo series alloy [for example, a HASTELLOY (registered trademark) C series (e.g., HASTELLOY C-276 (registered trademark) and HASTELLOY C-2(registered trademark)) manufactured by Haynes International; MAT21 manufactured by Mitsubishi Materials Corporation; and INCONEL (registered trademark) 625 manufactured by Inco Alloys International]; a Ni-Cr series or Ni-Cr-Fe series alloy [for example, MA276, MA625, MA600, MA-B2, MA-22, MA PLASTHARD S and MA PLASTHARD B-2, which are manufactured by Mitsubishi Materials Corporation; and INCONEL (registered trademark) 600 and 690 and INCOLOY 800 and 825, which are manufactured by Inco Alloys International].

The contact of the metallic unit as described above with the reaction mixture or the stream containing a carboxylic acid corrodes and elutes a metal component (for example, a metal other than Zr) constituting the unit to contaminate a product carboxylic acid with the eluted metal component. Zr is not substantially corroded in practical cases, while Ti may practically be eluted by corrosion. For example, Ti is sometimes used for a fixed-bed tower reactor or a fluidized-bed reactor.

The reactor is practically made of a nickel alloy (for example, the foregoing corrosion-resistant nickel alloy, in particular, a nickel alloy having a high corrosion resistance to an acid or an oxidizing atmosphere or the like), metal zirconium, a zirconium alloy, metal titanium, or a titanium alloy. Among these metals or alloys, a corrosion-resistant nickel alloy and metal zirconium are particularly preferred. In particular, for an industrial scale, a reactor made of a high-class metal (such as metal zirconium or metal titanium) is sometimes used.

Moreover, the peripheral unit of the reactor may be made of a nickel alloy, zirconium or an alloy thereof, or titanium or an alloy thereof. The peripheral unit is practically made of an iron alloy such as a stainless steel, in particular, an iron alloy having a high corrosion resistance to an acid or an oxidizing atmosphere [for example, NAS254N, NAS354N, NAS185N and NAS155N, which are manufactured by Nippon Yakin Kogyo Co., Ltd. ; andaFe-Cr-Ni series austenitic stainless steel (e.g., SUS304, SUS316L)].

Moreover, if necessary, an inner wall of the unit (e.g., the reaction system and the peripheral unit thereof) may be lined or covered (for example, lined with a glass, and lined with a fluorine-containing resin) or may be cladded with the above-exemplified nickel alloy, metal zirconium or alloy thereof, metal titanium or alloy thereof, metal tantalum or alloy thereof (e.g., a metal alloy containing tantalum), or others.

Moreover, since the carbonylation reaction system is an exothermic reaction system, the reactor may be equipped with a heat-removable (or heat-removing) or cooling unit (e.g., a jacket) for controlling a reaction temperature.

The withdrawing position of the stream (crude reaction liquid) containing the carboxylic acid in the reaction system is not particularly limited to a specific one as far as the liquid reaction mixture can be withdrawn appropriately according to the species of the reaction system or other conditions. In order to reduce the contamination with the metal-containing impurity and other impurities, although the carboxylic acid stream is practically withdrawn from a port situated in the upper part (e.g., upper half) of the liquid-phase of the reaction system, the metal-containing impurity are inevitably present in the withdrawn carboxylic acid stream. The carboxylic acid stream containing the metal-containing impurity withdrawn from the reaction system in such a manner is fed to the succeeding first flash system.

The space time yield (STY) of the objective carboxylic acid in the reaction system may be, for example, about 5 to 50 mol/L·h, preferably about 10 to 40 mol/L·h, and more preferably about 12 to 35 mol/L·h.

### [First flash system or step]

The stream (crude reaction liquid) containing the carboxylic acid withdrawn from the reaction system is fed to the first flash system and separated into a volatile (highly volatile) component (lower boiling point component) and a less-volatile or non-volatile component (higher boiling point component) by vaporization. In more detail, in the first flash system, the crude reaction liquid is separated into the highly volatile component (lower boiling point component) vapor and the liquid less-volatile or non-volatile component (higher boilingpoint component) containing the metal-containing impurity by the vapor-liquid equilibrium, and the metal-containing impurity contained in the liquid component may for example be mainly the metal catalyst and the metal-containing catalyst stabilizer such as an alkali metal halide, as well as a metal-containing component produced by corrosion of a metallic unit among the members or units such as the reactor, various lines, and the peripheral equipment).

The first flash system is a unit mainly designed for separating the metal component forming the catalyst system (e.g., the metal catalyst and/or the alkali metal halide). Thus it is sufficient that the first flash system can separate the metal component (the metal-containing impurity). The first flash system may comprise a commonly used evaporation apparatus such as a mixing tank (e.g., a mixing tank equipped with a jacket) or an evaporator (e.g., various evaporators such as a natural circulation evaporator, a forced circulation evaporator, an external heating tube evaporator, a long-tube vertical evaporator, an agitated-film evaporator, a coil evaporator, or a plate evaporator) or may comprise a conventional flash equipment (or apparatus) usingaflashphenomenon (flash evaporation), for example, a flash distillation column and a flash evaporator. The method of the flash evaporation may include, for example, an open-channel flow method and a spray flash evaporation method. Among the flash distillation columns and evaporators, the flash distillation column is practically used in the light of easy recycling of the separated metal component to the reaction system. Moreover, the first flash system may be provided with a reboiler or a jacket or may be provided with a conventional heating tube (for example, an external circulation heating tube).

In the first flash system, the vapor component and the liquid component may be separated from each other with or without heating. For example, in adiabatic flash, the reaction mixture may be separated into the vapor component and the liquid component without heating and with reduced pressure, and in thermostatic flash, the reaction mixture may be separated into the vapor component and the liquid component with heating and reduced pressure. The crude reaction liquid (the carboxylic acid-containing stream fed from the reaction system) may be separated into the vapor and the liquid by combining these flash conditions.

The feeding position of the crude reaction liquid (the carboxylic acid stream fed from the reaction system) to the first flash system is not particularly limited to a specific one, and can suitably be selected, e.g., depending on the species of the first flash system. For example, in a case where the flash distillation column is used, the crude reaction liquid is practically fed to the upper half (upper part) of the flash distillation column.

The first flash system (step) may be a single flash step or may be a combination of a plurality of flash steps.

The stream containing the carboxylic acid to be fed to the first flash system is not limited to the crude reaction liquid withdrawn from the reaction system and may be a carboxylic acid stream containing a metal-containing impurity. The stream (mixture) may contain a metal-containing impurity, a carboxylic acid, and other impurities.

The temperature of the first flash system or step (e.g., a flash distillation) may for example be about 30 to 250°C, preferably about 50 to 220°C (e.g., about 60 to 200°C), and more preferably about 80 to 180°C. Moreover, the pressure of the first flash system or step may be about 5 to 3,000 kPa (e.g., about 10 to 2,000 kPa), preferably about 20 to 1,500 kPa (e.g., about 30 to 1200 kPa), and more preferably about 40 to 1,000 kPa (e.g., about 50 to 800 kPa) in terms of absolute pressure.

The temperature and the pressure conditions of the first flash system (step) can suitably be combined from the foregoing ranges, depending on the species of the flash method (vacuum flash or thermostatic flash). For example, in a case where a carboxylic acid stream containing acetic acid formed by the reaction of methanol with carbon monoxide is subjected to the first flash distillation system, the temperature and the pressure (absolute pressure) may be, for example, about 70 to 200°C (preferably about 75 to 190°C) and about 80 to 400 kPa (preferably about 100 to 200 kPa), respectively.

Each of the material of the first flash system (such as a flash distillation column or an evaporator) and the material of the peripheral unit (for example, various lines communicating to the first flash system, e.g., a line for coupling the first flash system with another unit, such as a feed line or a stream withdrawn or discharge (distilling-off) line) of the first flash system may include a conventional material, for example, a glass, a metal, and a ceramic. The material usually includes a metal, for example, the above-exemplified metal or alloy, e.g., an iron alloy (such as a stainless steel), nickel or a nickel alloy, zirconium or a zirconium alloy, and titanium or a titanium alloy and practically includes a corrosion-resistant metal or alloy (e.g., a corrosion-resistant iron alloy, a nickel alloy such as HASTELLOY B-2 (registered trademark), metal zirconium or an alloy thereof, and metal titanium or an alloy thereof).

The liquid containing the higher boiling point component (e.g., a metal-containing impurity such as a metal component forming the catalyst system) separated in the first flash system may directly be collected and wasted or may be reused for other applications. The liquid is usually recycled to the reaction system. Moreover, if necessary, the liquid may be subjected to a separation treatment for separating a useful component (e.g., a constituent component of the catalyst system) prior to the recycling to the reaction system. Further, if necessary, the liquid may be heated or cooled before the liquid is fed (or recycled) to the reaction system.

The withdrawing position of the liquid containing the metal-containing impurity in the first flash system is not particularly limited to a specific one. The liquid ispracticallywithdrawnfromthe lowerpart (particularly, the bottom) of the flash system. For example, for the flash distillation column, the liquid is usually withdrawn from the bottom of the column.

The fraction [a fraction containing a volatile component (lower boiling point component) such as a separated carboxylic acid] vaporized in the first flash system contains an objective carboxylic acid [a carboxylic acid having "n+1" carbon atoms (e.g., acetic acid), corresponding to an alkanol having "n" carbon atom (s) (e.g., methanol)], and a co-catalyst (such as hydrogen iodide or methyl iodide), an ester of a raw material alkanol with a product carboxylic acid (e.g., methyl acetate), water, traces of by-products [for example, a carboxylic acid (e.g., a carboxylic acid having "n" carbon atom (s) (such as formic acid) and a carboxylic acid having "n+2" or more carbon atoms (such as propionic acid or crotonic acid)); an ester of such a carboxylic acid with a raw material alkanol (such as methyl propionate or methyl crotonate); an aldehyde having "n+1" or more carbon atoms (such as acetaldehyde, propionaldehyde, crotonaldehyde, or 2-ethylcrotonaldehyde); and an iodide (e.g., a C₂₋₁₅alkyl iodide (in particular, a C₂₋₁₂alkyl iodide) such as pentyl iodide, hexyl iodide, or decyl iodide)], or other impurities [for example, a higher boiling point impurity (such as a metal-containing impurity or a higher boiling point carbonyl impurity) produced by corrosion of at least one member selected from the group consisting of the reaction system, the flash system, and the line coupling these units] . For that reason, the fraction vaporized in the first flash system is further subjected to a distillation system to give a further purified objective carboxylic acid.

In the first flash system, it is preferable to conduct the flash evaporation while maintaining a lower linear velocity of the vaporized phase (that is, to extend or enlarge the inner diameter of a section or site through which the vapor in the flash system passes) in order to prevent the contamination of the objective carboxylic acid with an entrained impurity (e.g., a higher boiling point impurity). Moreover, a demister or a vapor-liquid separator may be installed. The linear velocity may be about 0.1 to 3 m/s, preferably about 0.2 to 2 m/s, and more preferably about 0.3 to 1.5 m/s.

### [Distillation system (non-flash distillation system) or distillation step]

The fraction vaporized in the first flash system is further fed to a distillation system. In the distillation system, the fraction is separated into an impurity and a stream mainly containing a carboxylic acid (objective carboxylic acid).

It is sufficient that the distillation system comprises at least one distillation apparatus (e.g., a distillation column) or distillation step. The distillation system may be a single distillation system (one distillation apparatus or distillation step) or may be a distillationsystem(multi-stage distillation system) having a combination of a plurality of distillation systems. Because of industrial point or cost, it is usually more preferable that the number of distillation apparatuses or steps be smaller. The distillation system may usually comprise 1 to 3 distillation apparatuses or steps, and more preferably 1 or 2 distillation apparatuses or steps.

The distillation system is not particularly limited to a specific one as far as the concentration (purity) of the obj ective carboxylic acid in the carboxylic acid stream from the distillation system can be larger than the concentration (purity) of the obj ective carboxylic acid in the stream fed to the distillation system. The distillation system comprises a conventional non-flash distillation apparatus (for example, a distiller or a distillation column), or others. In the distillation system, it is sufficient that an impurity having a boiling point lower than the boiling point of the objective carboxylic acid [a lower boiling point impurity (including an impurity having an azeotropic point lower than the boiling point of the objective carboxylic acid)] can be separated from the objective carboxylic acid. The lower boiling point impurity and a higher boiling point impurity having a boiling point higher than the boiling point of the objective carboxylic acid may be separated from the objective carboxylic acid.

The non-flash distillation apparatus may include, for example, a distiller such as a distillation still (or distillation flask), and a distillation column such as a plate column (e.g., a perforated plate column, a bubble-cap column, a Kittel tray column, a uniflux tray column, and a ripple tray column) or a packed column. For the distillation system containing a plurality of distillation apparatuses, the distillation system may comprise a combination of the same kind of distillation apparatuses (for example, a combination of a first bubble-cap column and a second bubble-cap column) or may comprise a combination of a different kind of distillation apparatuses [for example, a combination of a different kind of plate columns (e.g., a combination of a bubble-cap column and a perforated plate column), and a combination of a plate column (such as a bubble-cap column) and a packed column]. Among these distillation apparatuses, the distillation column is practically used.

The distillation temperature and pressure in the distillation system can suitably be selected depending on the condition such as the species of the objective carboxylic acid and the species of the distillation column, or the removal target selected from the lower boiling point impurity and the higher boiling point impurity according to the composition of the stream fed from the first flash system. For example, in a case where the purification of acetic acid is carried out by the distillation column, the inner pressure of the distillation column (usually, the pressure of the column top) may be 0.01 to 1 MPa, preferably about 0.02 to 0.7 MPa, and more preferably about 0.05 to 0.5 MPa in terms of gauge pressure. Moreover, the distillation temperature (for the distillation column, the inner temperature of the column (usually, the temperature of the column top)) can be controlled by adjusting the inner pressure of the apparatus (e.g., the inner pressure of the column), and may be, for example, about 20 to 200°C, preferably about 50 to 180°C, and more preferably about 100 to 160°C.

Moreover, in a case where the plate column or the packed column is used as the distillation system, the theoretical number of plates is not particularly limited to a specific one, and, depending on the species of the component to be separated, may be about 2 to 80, preferably about 5 to 60, and more preferably about 7 to 50 (e.g., about 10 to 40).

In the distillation system, the reflux ratio may be, for example, selected from about 0.01 to 3, 000 (e.g., about 0.05 to 1000), preferably about 0.07 to 500 (e.g., about 0.1 to 100), more preferably about 0.2 to 50 (e.g., about 0.25 to 10), and particularly about 0.3 to 5 (e.g., about 0.35 to 2). For the plate column or the packed column, the reflux ratio may be reduced by increasing the theoretical number of plates.

When the distillation apparatus (such as the distillation column) is used, a stream containing the lower boiling point impurity is withdrawn from the upper part of the distillation apparatus (e.g., the top of the column). Thus a stream containing the objective carboxylic acid may be withdrawn as a side cut stream (side stream) from the distillation column by side cut or may be withdrawn from the bottom of the column. Since the higher boiling point impurity tends to remain in the lower part of the distillation column (e.g., the bottom of the column), the withdrawing of the stream containing the carboxylic acid by side cut is advantageous in terms of efficient separation of the stream from the higher boiling point impurity. Moreover, the stream (e.g., a bottom liquid) containing the higher boiling point impurity may be collected from the lower part (such as the bottom) of the distillation apparatus. When the distillation column is used, the feeding position of the stream to be fed from the first flash system to the distillation column (the position (height) of the feed port to the distillation column) is not particularly limited to a specific one. From the viewpoint of efficient separation of the lower boiling point impurity and the higher boiling point impurity from the objective carboxylic acid, the feeding position or port of the stream is practically positioned at a lower part (or a lower plate) than the withdrawing port of the stream containing the lower boiling point impurity and an upper part (or a upper plate) than the withdrawing port (collecting port) of the stream containing the higher boiling point impurity.

The stream containing the higher boiling point impurity collected from the lower part of the distillation apparatus (e.g., a distillation column) may be wasted directly or may be recycled to the reaction system or the first flash system. Moreover, prior to the recycling, an unnecessary component (e.g., a component deteriorating in the quality of the objective carboxylic acid) may be removed from the stream. The unnecessary component may include, for example, a carbonyl impurity having a boiling point higher or lower than the boilingpoint of the obj ective carboxylic acid [e.g., a carboxylic acid having "n+2" carbon atoms (propionic acid, crotonic acid and others, in a case where acetic acid is the objective carboxylic acid); a ketone such as acetone; an aldehyde such as acetaldehyde, propionaldehyde, crotonaldehyde, or 2-ethylcrotonaldehyde; an ester such as methyl acetate, methyl propionate, or methyl crotonate (e.g., an ester of a raw material alcohol with a product or by-product carboxylic acid)].

The stream containing the lower boiling point impurity withdrawn from the upper part of the distillation apparatus may be collected directly or may be recycled to the reaction system or the distillation apparatus. Prior to the recycling, the stream containing the lower boiling point impurity may be condensed by a condenser or other means, or may be condensed by a condenser or other means to form a plurality of separated phases in a decanter (or by decantation or other means). Moreover, after the decantation, the component to be recycled to the reaction system or the distillation apparatus may be selected or prepared depending on the species of the components of each phase. For example, when the condensate of the stream is separated into an organic phase and an aqueous phase by decantation, a portion of the aqueous phase may be returned to the distillation column, and the remainder of the aqueous phase and the organic phase may be recycled to the reaction system. Alternatively, a portion of the organic phase may be returned to the distillation column and the remainder of the organic phase and the aqueous phase may be recycled to the reaction system. Prior to the recycling, an unnecessary component (for example, an aldehyde such as acetaldehyde) may be separated from the stream (fraction) containing the lower boiling point impurity separated in the distillation system. The stream may be recycled directly without separation of the unnecessary component. The stream containing the lower boiling point impurity is preferably subjected to an aldehyde separation system for separating or removing an aldehyde contained in the lower boiling point impurity after the stream is optionally condensed by a condenser and/or phase-separated by decantation.

In a case where the stream containing the lower boiling point impurity is recycled to the distillation apparatus (e.g., a distillation column), a rectification action is preferably used in order to increase the separation effect of the lower boiling point impurity. Thus it is preferable that the stream to be recycled to the distillation apparatus be a liquid form (that is, in at least a condensed state). In particular, in order to improve the countercurrent contact efficiency of the stream with an ascending gaseous component in the distillation apparatus, the liquid stream is preferably returned (ref luxed) to the upper part of the distillation apparatus.

The material of each member or unit associated with the distillation system [for example, the distillation system and the peripheral unit thereof, e.g., a unit (such as a condenser or a decanter), and various lines, each communicating to the distillation system (such as a line for coupling units)] is not particularly limited to a specific one. As the material, a conventional material (e.g., a glass, a metal, and a ceramic) can be used. According to the present invention, the material of the foregoing distillation system and that of the member or unit are practically a metal [for example, the above-exemplified iron alloy (e.g., a stainless steel), the above-exemplified nickel or nickel alloy (e.g., HASTELLOY B-2 (registered trademark)), metal zirconium or an alloy thereof, metal titanium or an alloy thereof]. Even in a case where the metal-containing impurity is produced by corrosion of such a metallic distillation system and/or unit, the present invention allows efficient separation of the metal-containing impurity and production of a high-purity product carboxylic acid.

As described above, among the impurity-containing streams from the distillation system, the stream containing the lower boiling point impurity contains an impurity having a boiling point lower than the boiling point of the objective carboxylic acid, and the stream containing the higher boiling point impurity contains an impurity having a boiling point higher than the boiling point of the obj ective carboxylic acid. For example, in a case where the objective carboxylic acid is acetic acid, the lower boiling pointimpurity contains,for example,aco-catalyst (such as hydrogen iodide or methyl iodide), a trace of an alkyl iodide (e.g., a C₂₋₃alkyl iodide), and a lower boiling point carbonyl impurity [e.g., formic acid, a carboxylic acid ester (such as methyl formate, methyl acetate, or methyl propionate), water, and an aldehyde (such as acetaldehyde, propionaldehyde, or crotonaldehyde)], and the higher boiling point impurity contains, for example, an alkyl iodide (e.g., a C₄₋₁₅alkyl iodide), a higher boiling point impurity (e.g., a metal-containing impurity) produced by corrosion of various units constituting the production apparatus according to the present invention [for example, a metallic unit (e.g., at least one unit selected from the group consisting of a reaction system, a flash system, a distillation system, a condenser, a decanter, and each of lines for coupling these units)], and a higher boiling point carbonyl impurity (e.g., a carboxylic acid higher than acetic acid, such as propionic acid or crotonic acid).

According to the present invention, it is sufficient that the distillation system can effectively separate the lower boiling point impurity (and higher boiling point impurity) and the obj ective carboxylic acid, as described above. The distillation system has at least one distillation apparatus (e.g., a distillation column). Moreover, the distillation system may have a plurality of distillation apparatuses (e.g., distillation columns). For example, the separation of the impurity from the objective carboxylic acid may be carried out by the distillation system containing one distillation apparatus (e.g., a distillation column). Depending on the composition of the stream to be fed from the flash system, the separation of the impurity from the objective carboxylic acid may be carried out by the distillation system containing two distillation apparatuses [two distillation apparatuses (e.g., distillation columns) having a first distillation apparatus (e.g., a first distillation column) and a second distillation apparatus (e.g., a second distillation column)]. In a preferred embodiment, the distillation system contains at least one distillation column; the distillation system may contain two distillation columns, i.e., a first distillation column and a second distillation column.

In a case where the distillation system comprises a single distillation column, the withdrawing position (the height of the withdrawing port) of the objective carboxylic acid is preferably positioned at a lower part (or a lower plate) than the position of the foregoing feed port, in terms of efficient separation of the lower boiling point impurity from the objective carboxylic acid.

Moreover, in a case where the impurity and the obj ective carboxylic acid are separated by two distillation apparatuses, the purity of the objective carboxylic acid may further be improved by separating a stream containing the lower boiling point impurity, a stream containing the higher boiling point impurity, and a stream containing the objective carboxylic acid in a first distillation apparatus (e.g., a distillation column), as the same manner as in the single distillation column; and then feeding the steam containing the objective carboxylic acid to a second distillation apparatus (e.g., a distillation column) to separate the objective carboxylic acid from an impurity inevitably contained in the stream [in particularly, for example, an impurity having a boiling point close to the boilingpoint of the obj ective carboxylic acid (e.g., water and crotonaldehyde, in a case where the objective carboxylic acid is acetic acid)] in the second distillation apparatus.

In a case where two distillation apparatuses are used, the condition of each distillation apparatus, the feeding position, collecting or withdrawing position of the stream, the recycling of the collected stream or component, and others can suitably be selected (or conducted) as similar to the conditions of the single distillation column.

In a case where two distillation apparatuses are used, it is preferable that the lower boiling point impurity and the higher boiling point impurity be separated from the stream containing the carboxylic acid by the first distillation apparatus (first distillation column) as described above, and then the stream containing the carboxylic acidbe fed to the second distillation apparatus (second distillation column) to separate into the stream containing the lower boiling point impurity and the stream containing the objective carboxylic acid by the second distillation apparatus. In this aspect, in the second distillation apparatus, the lower boiling point impurity may be withdrawn from the upper part of the distillation apparatus (e.g., the top of the column), and the stream containing the objective carboxylic acid may be collected from the lower part of the distillation apparatus (e.g., the bottom of the column) or may be collected as a side cut stream by side cut.

In a case where two distillation apparatuses are used, the relationship between the feeding position and the withdrawing position (collecting position) of the stream containing the objective carboxylic acid in each distillation column can suitably be selected depending on the composition of the stream to be fed to each distillation column or the target impurity of the separation in each distillation column. For example, in the second distillation column, the objective carboxylic acid stream may be collected from a collecting port (or withdrawing port) below a feed port of the stream from the first distillation column. Alternatively, for example, the stream containing the objective carboxylic acid in the first distillation column is collected (withdrawn) fromacollectingport (withdrawingport) below a feed port of the stream from the first flash system and is fed to the second distillation column, the objective carboxylic acid in the second distillation column may be collected from a collecting port (withdrawing port) below an inlet port for feeding the stream to the second distillation column and above an outlet port (withdrawing port) of the higher boiling point impurity. Alternatively, the stream containing the objective carboxylic acid in the first distillation column is collected (withdrawn) from a collecting port (withdrawing port) above a feed port for feeding the stream to the first distillation column and fed to the second distillation column, the objective carboxylic acid in the second distillation column may be collected from the collecting port (with drawing port) below a feed port for feeding the stream to the second distillation column and may be collected from the bottom of the second distillation column (in other words, it is not necessary to separate the higher boiling point impurity in the second distillation column).

Also, in a case where the second distillation column is mainly designed for removing the lower boiling point impurity, the temperature, the pressure, the theoretical number of plates, the reflux ratio, and others, in the second distillation column can be selected from the ranges as exemplified above. These conditions may be the same as those in the first distillation column. The conditions in the first distillation column and those in the second distillation column may suitably be different from each other depending on the composition of the stream to be fed to the second distillation column, the desired purity of the objective carboxylic acid to be collected from the second distillation column, or others.

For example, in a case where the purification of acetic acid is carried out by a plate column, the inner pressure (usually, the pressure of the column top) of each of the first and second distillation columns can suitably be selected from the range as the same as the above-mentioned inner pressure of the distillation column. The inner pressure of the first distillation column may for example be about 0.07 to 0.4 MPa, preferably about 0.08 to 0.3 MPa, and more preferably about 0.09 to 0.2 MPa in terms of gauge pressure. The inner pressure (usually, the pressure of the column top) of the second distillation column may be about 0.07 to 0.4 MPa, preferably about 0.09 to 0.3 MPa, and more preferably about 0.1 to 0.25 MPa in terms of gauge pressure. The inner temperature (usually, the temperature of the column top) of the distillation column can be controlled by adjusting the inner pressure. For the first distillation column, the inner temperature may for example be about 30 to 180°C, preferably about 60 to 150°C, and more preferably about 100 to 130°C; for the second distillation column, the inner temperature may for example be about 70 to 200°C, preferably about 100 to 180°C, and more preferably about 110 to 160°C.

Moreover, in a case where each of the first and the second distillation columns is a plate column or a packed column, the theoretical number of plates of each distillation column can be selected from the range as exemplified in the aforementioned theoretical number of plates of the distillation system. The theoretical number of plates of the first distillation column may for example be about 2 to 30, preferably about 3 to 20, and more preferably about 5 to 15; the theoretical number of plates of the second distillation column may for example be about 5 to 50, preferably about 10 to 40, and more preferably about 15 to 35.

Moreover, the reflux ratio in each of the first and the second distillation columns can suitably be selected from the range as exemplified in the aforementioned reflux ratio in the distillation system. The reflux ratio in the first distillation column may for example be selected from about 0.01 to 10, preferably about 0.05 to 5 (e.g., about 0.07 to 3), and more preferably about 0.1 to 1.5; the reflux ratio in the second distillation column may for example be selected from about 0.01 to 30, preferably about 0.05 to 20, and more preferably about 0.1 to 10 (e.g., about 0.5 to 7).

The stream containing the lower boiling point impurity withdrawn from the upper part of the second distillation apparatus may be collected directly or may be recycled to the reaction system and/or the distillation apparatus, as the same manner as in the single distillation column. In a case where the stream is recycled to the reaction system, the stream containing the lower boiling point impurity from the first distillation apparatus and the stream containing the lower boiling point impurity from the second distillation apparatus may be joined (or combined) and recycled to the reaction system, or may be recycled separately to the reaction system without being joined (or combined). As mentioned above, prior to recycling to the reactor, an unnecessary component (e.g., an aldehyde such as acetaldehyde) may be separated from the stream containing the lower boiling point impurity from the second distillation apparatus. In a case where the streams, each containing the lower boiling point impurity, from the first and the second distillation apparatuses are joined, the unnecessary component may be separated from each stream before these streams are joined or may be separated after these streams are joined.

The material of each unit associated with the second distillation apparatus (for example, a line for coupling the first distillation apparatus with the second distillation apparatus, and other peripheral units) is not particularly limited to a specific one, and may include a conventional material, for example, an iron alloy (such as a stainless steel), nickel or a nickel alloy (such as HASTELLOY B-2 (registered trademark)), metal zirconium or an alloy thereof, and metal titanium or an alloy thereof.

### (Aldehyde separation system)

In a case where the lower boiling point impurity separated in the distillation system is recycled (returned) to the reaction system, an aldehyde (such as acetaldehyde) may be removed from the stream containing the lower boiling point impurity by subjecting the stream to an aldehyde separation system before the stream is recycled to the reaction system, as described above.

As the aldehyde separation system, a conventional separation means may be used, for example, a conventional distillation apparatus (e.g., the distillation apparatus as exemplified above) or a conventional absorption apparatus. In particular, a distillation column (for example, a plate column, a packed column, and a flash distillation column), an absorber (absorption apparatus) or an extractor (extraction apparatus, for example, a column (ortower) typeandatank (orvessel) type extractor). For the distillation column, an aldehyde can be removed efficiently by adding an aldehyde-absorbing solvent (e.g., water) or an organic solvent, efficiently separating an aldehyde, (for example, an ether, e.g., a dialkyl ether such as dimethyl ether (DME)) to the upper part of the column so as to bring an aldehyde-containing gaseous stream ascending from the lower part of the column into countercurrent-contact with the solvent. Moreover, for the absorber or the extractor, it is sufficient that an aldehyde-absorbing solvent (e.g., water) is mixed or contacted with the stream containing an aldehyde. The absorber or the extractor may be a column type (for example, a Karr column, a spray column, a packed column, a perforated plate column, a baffled column, and a pulse column) or may be a tank type (e.g., an absorption or extraction tank equipped with a stirrer).

The temperature of the aldehyde separation system and the pressure thereof are not particularly limited as far as an aldehyde is separable from other lower boiling point impurities (in particular, an alkyl halide such as methyl iodide). The temperature and the pressure can be selected depending on the species of the aldehyde and other lower boiling point impurities, and the species of the separation system (e.g., adistillation column), or others.

For example, in the purification of acetic acid, in a case where the aldehyde separation system is a distillation column or an absorption column (in particular, such as a plate column or a packed column), the pressure of the column top may be about 10 to 1, 000 kPa, preferably about 50 to 700 kPa, and more preferably about 80 to 500 kPa (e.g., about 100 to 300 kPa) in terms of absolute pressure. Moreover, the inner temperature (the temperature of the column top) may for example be about 10 to 150°C, preferably about 20 to 130°C, and more preferably about 30 to 100°C.

In a case where the aldehyde separation system is a distillation column or an absorption column (e.g., a plate column or a packed column), the theoretical number of plates may for example be about 5 to 80, preferably about 6 to 70, and more preferably about 8 to 65 (e.g., about 10 to 60). Moreover, the reflux ratio canbe selected from the range of about 1 to 1,000, preferably about 10 to 800, and more preferably about 50 to 600 (e.g., about 100 to 600) depending on the theoretical number of plates.

In the aldehyde separation system, in a case where the solvent (e.g., water) is brought into contact with the aldehyde-containing stream, the temperature of the solvent to be fed to the separation system is not particularly limited to a specific one, and may for example be about 0.1 to 50°C, preferably about 1 to 30°C, and more preferably about 5 to 20°C (in particular, about 7 to 15°C).

The removal of the aldehyde contained in the stream recycled from the distillation system to the reaction system can inhibit the formation of by-product carboxylic acid having "n+2" or more carbon atoms in the reaction system, so that the concentration of the carboxylic acid having "n+2" or more carbon atoms in the stream containing the carboxylic acid to be fed to the distillation system can drastically be reduced. In a case where the reaction system is a reaction system of an alkanol having "n" carbon atom(s) with carbon monoxide, it is useful to remove the aldehyde having "n+1" or more carbon atoms [for example, an aldehyde having 2 or more carbon atoms (such as acetaldehyde) for a reaction system of methanol with carbon monoxide]. Further, the removal of the aldehyde effectively inhibits the formation of a carboxylic acid having carbon atoms more than that of the objective carboxylic acid [for example, a carboxylic acid having 3 or more carbon atoms (such as propionic acid) in a case where the objective carboxylic acid is acetic acid)]. Thus even in the stream to be fed to the distillation system, the concentration of the carboxylic acid having "n+2" or more carbon atoms can be reduced within the quality of the product standard (or criteria) of the objective carboxylic acid. Accordingly, in a case where the distillation system comprises a plurality of distillation apparatuses (e.g., distillation columns), the carboxylic acid having "n+2" or more carbon atoms may be separated by the second distillation apparatus. Even if the carboxylic acid having "n+2" or more carbon atoms is not separated positively, the concentration of the carboxylic acid from the second distillation apparatus is enough low. Thus there is no need to provide a further distillation apparatus (e.g., a third distillation apparatus) in order to further separate the carboxylic acid having "n+2" or more carbon atoms. Moreover, since the production amount of an unsaturated aldehyde having "n+2" or more carbon atoms (such as crotonaldehyde or 2-ethylcrotonaldehyde, in a case where the objective carboxylic acid is acetic acid) can be reduced, the potassium permanganate test value of the product carboxylic acid can drastically be improved without a special treatment (such as an ozonation). For example, the product carboxylic acid can have a potassium permanganate test value, which is a product standard (or criteria), of not less than 120 minutes. Moreover, since the formation of an alkyl iodide can also be reduced, the load on the ion exchange resin can be reduced in a further treatment with the ion exchange resin.

The material of the aldehyde separation system or a peripheral unit thereof (e.g., a line for communicating to the aldehyde separation system) is not particularly limited to a specific one, and may include a conventional material, for example, a glass, a metal [for example, the above-exemplified metal or metal alloy (e.g., a corrosion-resistant metal or alloy), e.g., an iron alloy such as a stainless steel, a nickel alloy such as HASTELLOY B-2 (registered trademark), metal zirconium, a zirconium alloy, metal titanium, or a titanium alloy)], and a ceramic.

For example, the industrial treatment method in the aldehyde separation system may include as follows.
(1) a method in which an offgas withdrawn from a condenser through a distillation system (for example, a first and/or second distillation system) is fed to the lower part of an aldehyde-removing column (aldehyde-absorption column) and is brought into contact with water in the column to absorb an aldehyde (e.g., acetaldehyde) contained in the offgas to the water, and the resulting aqueous solution is withdrawn as a waste liquid from the bottom of the aldehyde-absorption column;
(2) a method in which a portion of a condensate (an organic phase and/or an aqueous phase) obtained from a condenser through a distillation column (e.g., a first distillation system) is fed to the lower part of an aldehyde-removing column, a stream containing methyl iodide, acetic acid, methyl acetate, and water, and others is withdrawn from the bottom of the column and is recycled to a reactor, a stream withdrawn from the top of the aldehyde-removing column is refluxed at the upper part of the aldehyde-removing column, and an aldehyde is separated from the top of the column; (3) a method which is the same manner as in the method (2) except that the stream from the bottom of the aldehyde-removing column is introduced into an extraction unit (e.g., an aldehyde extraction column) without recycling to the reactor, is subjected to extraction with water for extracting an aldehyde, and is then introduced into a distillation column for removing the aldehyde. In the method (3), it is not necessary to separate an aldehyde from the top of the aldehyde-removing column while refluxing the stream withdrawn from the top of the aldehyde-removing column at the upper part of the column. Moreover, another method may include (4) a method in which a liquid and/or aldehyde-containing process liquid withdrawn from a distillation system (e.g., a first distillation system) is fed to an extraction unit (e.g., the lower part of an aldehyde extraction column), the aldehyde (e.g., acetaldehyde) is extracted with water, the extracted aldehyde aqueous solution is fed to another distillation column, the aldehyde (e.g., acetaldehyde) is separated and removed from the top of the column and a bottom liquid (raffinate) from the column is recycled to a reactor or wasted. As the extraction unit, there may be used an extraction column [for example, a packed column, e.g. , a packed column packed with a packing (e.g., a commonly used irregular packing (such as raschig ring, cascade ring) and a commonly used regular packing (such as sulzer pack)), a baffle column, and a perforated plate column]. The theoretical number of plates in the extraction unit may be, for example, about 1 to 4 (preferably about 2 to 3). Moreover, the extraction unit may be a mixer-settler type apparatus (for example, an extraction apparatus provided with 1 to 4 couples (or sets) of a mixer and a settler) or others. Further, the extraction of the aldehyde with water and the distillation may be carried out by an extractive distillation column to separate the aldehyde.

[Removal of impurity (e.g., metal-containing impurity, carbonyl impurity (such as higher boiling point carbonyl impurity), and higher halide)]

According to the present invention, a high-purity carboxylic acid is produced by further feeding the stream mainly containing the carboxylic acid (objective carboxylic acid) separated in the distillation system to a second flash system or an adsorption system and by collecting a purified carboxylic acid from the second flash system or the adsorption system. By subjecting the stream to the second flash system or the adsorption system, a metal-containing impurity, and a higher boiling point carbonyl impurity and/or a higher iodide, and others can effectively be removed, and the resulting product carboxylic acid can be prevented from coloring and can achieve an markedly improved purity. In particular, the iodine ion removal efficiency can be increased by feeding the stream to the adsorption system without an oxidation treatment. Moreover, the potassium permanganate test value and/or the potassium bichromate test value of the product carboxylic acid can be improved significantly.

The metal-containing impurity may include (i) at least one member selected from the group consisting of a metal catalyst, a deactivated product thereof, a catalyst stabilizer, and a deactivated product thereof, and/or (ii) a metal-containing component (impurity) produced by corrosion of a metallic unit. In the second flash system or the adsorption system, the former entrained metal-containing impurity (i) may be removed. The second flash system or the adsorption system practically intends to separate the latter metal-containing impurity (ii).

The metallic unit may include a metallic member or unit among various members or units of the production apparatus according to the present invention, for example, at least one member selected from the group consisting of the above-exemplified various units, specifically, a unit (e.g., a reaction system, a first flash system, a distillation system, a condenser, a decanter, a second flash system, and an adsorption system), and lines for communicating to these units or coupling these units.

Among these units, at least one member selected from the group consisting of a reaction system, a first flash system, and a line (stream feed line) for coupling the reaction system with the first flash system may be made of a metal [for example, the above-exemplified metal or metal alloy (e.g., a corrosion-resistant metal or alloy), e.g., an iron alloy such as a stainless steel, a nickel alloy such as HASTELLOY B-2 (registered trademark), metal zirconium, a zirconium alloy, metal titanium, or a titanium alloy)], preferably a nickel alloy, zirconium or an alloy thereof, or titanium or an alloy thereof. Moreover, at least one member selected from the group consisting of a reaction system, a first flash system, a distillation system, a line (stream feed line) for coupling the reaction system with the first flash system, a line (stream feed line) for coupling the first flash system with the distillation system, and a line (stream feed line) for coupling the distillation system with the second flash system or the adsorption system may be made of a metal (in particular, a stainless steel, for example, an austenitic stainless steel).

Depending on the material of the unit, the species of the component contained in the stream, or other factors, the impurity (e.g., a metal-containing impurity) produced by corrosion of the metallic unit may include, for example, a compound containing various metal elements (e.g., an oxide and a halide such as an iodide or a chloride), and a salt (e.g., an inorganic acid salt such as a sulfate, a nitrate, or a phosphate, and an organic acid salt (e.g., a carboxylic acid salt) such as an acetate). The metal element may include, for example, an alkali metal such as lithium, sodium, or potassium; an alkaline earth metal such as beryllium, magnesium, or calcium; a light metal such as titanium or aluminum (e.g., mainly, a metal having specific gravity of less than 5), and a heavy metal (e.g., mainly, a metal having a specific gravity of not less than 5), for example, niobium, tantalum, chromium, molybdenum, manganese, iron, nickel, copper, zinc, and lead. The metal element may also include a transition metal element (e.g., a group 4 metal element of the Periodic Table such as titanium or zirconium; a group 5 metal element of the Periodic Table such as vanadium, niobium, or tantalum; a group 6 metal element of the Periodic Table such as chromium, molybdenum, or tungsten; a group 7 metal element of the Periodic Table such as manganese; a group 8 metal element of the Periodic Table such as iron or ruthenium; a group 9 metal element of the Periodic Table such as cobalt or rhodium; a group 10 metal element of the Periodic Table such as nickel, palladium, or platinum; and a group 11 metal element of the Periodic Table such as copper or silver), a typical metalelement (e.g., the above-exemplified alkali metal or alkaline earth metal element; a group 12 metal element of the Periodic Table such as zinc; and a group 13 metal element of the Periodic Table such as aluminum). The metal-containing impurity may contain a single metal element or a plurality of metal elements among these metal elements.

The metal-containing impurity practically contains the above-mentioned transition metal element and/or heavy metal element. In a case where a stainless steel or the like is used as the material of the metallic unit, the metal-containing impurity contains an iron element and may contain titanium, niobium, tantalum, manganese, chromium, molybdenum, nickel and/or aluminum together with the iron element. Moreover, in a case where a nickel alloy or the like is used as the material of the metallic unit, the metal-containing impurity practically contains nickel, and in addition, chromium, molybdenum, iron, tantalum, manganese, aluminum, an alkali metal, and/or an alkaline earth metal.

As the higher boiling point carbonyl impurity, there may be mentioned the above-exemplified higher boiling point carbonyl impurity (in particular, e.g., a carboxylic acid higher than the objective carboxylic acid).

Moreover, the higher halide may include a C₄₋₁₂alkyl halide (in particular, a C₄₋₁₂alkyl iodide) such as pentyl iodide, hexyl iodide, or decyl iodide.

### [Second flash system (or step)]

In the second flash system, the stream mainly containing the carboxylic acid fed from the distillation system is subjected to vaporization to separate into a volatile (highly volatile) component (that is, a stream of a purified objective carboxylic acid) and a less-volatile or non-volatile component (in particular, a higher boiling point impurity such as the above-mentioned metal-containing impurity, the higher boiling point carbonyl impurity, or the higher halide).

The second flash system is not particularly limited to a specific one. The conventional flash equipment or apparatus (such as a flash distillation column or a flash evaporator) as exemplified in paragraph of the first flash system may be used as the second flash system. Moreover, the second flash system may be provided with a reboiler or a jacket or may be provided with a conventional heating tube (for example, an external circulation heating tube) or others. The carboxylic acid stream to be fed to the second flash system has been highly purified of an impurity [(e.g., a lower boiling point (highly volatile) impurity and a higher boiling point (less-volatile or non-volatile) impurity), particularly a lower boiling point impurity] by the first flash system and the distillation system. Thus in the second flash system, it is sufficient that the flash evaporation of the objective carboxylic acid can remove the less-volatile or non-volatileimpurity (e.g., a metal-containing impurity, a higher boiling point carbonyl impurity, and a higher halide). In this respect, use of even a simple flash apparatus such as a flash evaporator achieves effective removal of the metal-containing impurity.

In the second flash system, the vapor component and the liquid component may be separated from each other with or without heating, as the same as in the first flash system. Moreover, the second flash system may be an adiabatic flash or a thermostatic flash.

The feeding position (the position or height of the feed port) of the carboxylic acid stream to the second flash system is not particularly limited to a specific one and can suitably be selected depending on the species of the flash system (apparatus), or others.

The temperature of the second flash system (e.g., a flash evaporator) may for example be about 50 to 250°C, preferably about 60 to 200°C, and more preferably about 80 to 180°C. Moreover, the pressure thereof may be about 5 to 1,000 kPa, preferably about 10 to 800 kPa, and more preferably about 20 to 500 kPa in terms of absolute pressure.

The temperature and the pressure of the second flash system can suitably be combined from the above ranges, depending on either vacuum flash or thermostatic flash. For example, the stream containing carboxylic acid from the distillation system may be subjected to the second flash system having a temperature of about 55 to 220°C (preferably about 65 to 190°C) and a pressure (absolute pressure) of about 10 to 1,000 kPa (preferably about 20 to 600 kPa) to vaporize the carboxylic acid for separating a higher boiling (less-volatile or non-volatile) impurity and the carboxylic acid from each other. For example, when the objective carboxylic acid is acetic acid, the temperature and the pressure (absolute pressure) may be, for example, about 70 to 200°C (preferably about 75 to 190°C) and about 20 to 600 kPa (preferably about 30 to 600 kPa), respectively.

The less-volatile or non-volatile component (e.g., a metal-containing impurity) separated by the second flash system may be collected directly from the lower part (or bottom) of the flash system (such as a flash distillation column or an evaporator) and wasted or may be reused for other applications.

In this way, in the second flash system, the metal-containing impurity (in particular, e.g., a metal-containing impurity produced by corrosion of the metallic unit),the higher boiling point carbonylimpurity, the higher halide, and others are highly removed, and a purified objective carboxylic acid can be obtained from the upper part (or top) of the second flash system.

### [Adsorption system (or step)]

In the adsorption system, a higher boiling point (less-volatile or non-volatile) impurity (e.g., the above-mentioned metal-containing impurity, the higher boiling point carbonyl impurity, and the higher halide) contaminating the stream mainly containing the carboxylic acid fed from the distillation system is adsorbed to or on an adsorbent to obtain a purified carboxylic acid. In more detail, the impurity is adsorbed on an adsorbent by bringing the stream mainly containing the carboxylic acid separated in the distillation system into contact with the adsorbent, without an oxidation treatment by an adsorbent (such as a solid strong oxidizer), thereby being separated from the carboxylic acid. Since the adsorption is different in separation principle from an ion exchange with an ion exchange resin, the adsorption system used in the present invention does not include a separation system (apparatus or step) withusing an ion exchange resin.

As the adsorbent, there may be used a conventional adsorbent. Depending on the species of the impurity contained in the carboxylic acid stream fed from the distillation system, the species of the adsorbent can suitably be selected. The adsorbent may also include a porous solid matter, for example, an activated carbon and a zeolite, and a metal oxide (e.g., a silica, an alumina, and a titania). The adsorbents may be used alone or in combination. Probably because a solid strong oxidizer (such as potassium bromate) as the adsorbent oxidizes an iodine ion in the stream containing the carboxylic acid to form iodine (I₂), which is not suitable for adsorption, the solid strong oxidizer is not effective in removing the iodine ion in the carboxylic acid stream. Thus, differently from the Patent Documents 3 and 4, the adsorption treatment of the present invention is conducted without an oxidation treatment and the adsorbent or the adsorption system does not contain a solid strong oxidizer or an adsorption column with a solid strong oxidizer.

For adsorbing an organic solvent, it is conventional to use an activated carbon, which has a strong affinity and a high adsorption even in the presence of water. The activated carbon is effective in adsorbing a sulfur compound or a metal compound and is widely used industrially. Moreover, the silica gel or the zeolite is particularly effective in adsorbing a highly polarsolvent. A porous solid, as the adsorbent, having a larger specific surface area or pore volume usually has a higher adsorption performance.

The form of the adsorbent is not particularly limited to a specific one and may include a powder, a particle (e.g., a bead), a flake, a fiber, a shaped form (e.g., a honeycomb), and others.

The adsorption unit (adsorption apparatus or adsorber) used for the adsorption system is not particularly limited to a specific one and may be a conventional adsorption apparatus, for example, a contact filtration apparatus, a fixed-bed adsorption apparatus, and a moving-bed adsorption apparatus. In order to continuously produce a purified carboxylic acid, use of a fixed-bed or moving-bed adsorption apparatus (in particular, a moving-bed adsorption apparatus) is advantageous.

The stream containing the carboxylic acid fed to the adsorption system may be a liquid or a gaseous form. The lower limit of the temperature of the adsorption system is a freezing point of an objective carboxylic acid having "n+1" carbon atoms (for example, the freezing point is 17°C for acetic acid). The upper limit thereof is a heat-resisting temperature of the adsorption system. The stream may usually be treated at a temperature of not higher than 200°C. For a continuous flow type adsorption system, the influence of the film diffusion resistance is usually reduced by suitably adjusting the flow rate of the stream passing through the adsorption system and/or the bed volume per hour (every hour) thereof. An economically advantageous condition can suitably be used depending on the characteristic of the adsorbent to be used, the species, characteristic and concentration of the impurity to be removed, and others.

### [Separation of other impurities]

The purified carboxylic acid stream obtained from the second flash system or the adsorption system may directly be used as a product carboxylic acid or may be subjected to a further separation system (separation step) for separating or removing a trace of an impurity. As the separation system, there may be used a conventional means, for example, a distillation means, an absorption means, an adsorption means, and an ion exchange means, depending on the species of the impurity to be removed.

In particular, the purified carboxylic acid stream obtained from the second flash system or the adsorption system sometimes contains a halide having a boiling point more than or close to the boiling point of the objective carboxylic acid; the halide may include an alkyl halide (for example, an alkyl iodide (e.g., a C₂₋₁₅alkyl iodide) such as pentyl iodide or hexyl iodide). Thus, in order to remove the halide, an ion exchange treatment may be carried out.

### (Ion exchange system or step)

In the ion exchange system, an impurity (mainly, the above halide) is removed from the stream containing the carboxylic acid from the second flash system or the adsorption system by treating the stream with an ion exchange resin.

The ion exchange resin is not particularly limited to a specific one as far as the ion exchange resin has a halide-removing capability. The ion exchange resin may include a conventional ion exchange resin, and may be an ion exchange resin (usually, a cation exchange resin) in which at least part of active sites (usually, e.g., an acidic group such as a sulfone group, a carboxyl group, a phenolic hydroxyl group, or a phosphone group) has been replaced with or exchanged for a metal.

The metal described above may include, for example, at least one member selected from the group consisting of silver, mercury, and copper. The cation exchange resin as a base may be a strongly acidic cation exchange resin or may be a slightly acidic cation exchange resin. The strongly acidic cation exchange resin (for example, a macroreticular ion exchange resin) is preferred.

In the ion exchange resin, for example, about 10 to 80% by mol, preferably about 25 to 75% by mol, and more preferably about 30 to 70% by mol, of the active sites may be exchanged for the metal described above.

As an ion exchange means (or apparatus) with the ion exchange system, there may be used a conventional ion exchange apparatus having an ion exchange resin inside thereof, for example, a packed column packed with an ion exchange resin, and a column provided with an ion exchange resin bed (for example, a bed having a granular resin) (guard bed).

In the ion exchange system, it is sufficient that the carboxylic acid stream can be brought into contact with the ion exchange resin. As the ion exchange resin, it is preferred to use at least the above-mentioned metal-exchanged ion exchange resin. Moreover, the metal-exchanged ion exchange resin may be used in combination with an ion exchange resin (such as a cation exchange resin, an anion exchange resin, or a nonion exchange resin) other than the above-mentioned metal-exchanged ion exchange resin. For the combination of these ion exchange resins, there may be used a mixture of both resins, an ion exchange system (such as a column) having each layer of these resins inside thereof, or an ion exchange system containing a unit (such as a column) provided with a metal-exchanged ion exchange resin and a unit (such as a column) provided with another ion exchange resin.

It is sufficient that the carboxylic acid stream from the second flash system or the adsorption system in the form of a gas or a liquid is brought into contact with at least the ion exchange resin. It is preferred to bring the stream in the liquid form into contact with the ion exchange resin. In particular, it is preferable that the carboxylic acid stream from the second flash system or the adsorption system be condensed and liquefied by a condenser or other means and the resulting liquid stream pass through the ion exchange resin.

During the contact of the carboxylic acid stream with the ion exchange resin (or the running of the carboxylic acid stream through the ion exchange resin), the carboxylic acid stream may be treated with the ion exchange resin under a warm or higher (or increased) temperature, if necessary. Moreover, the temperature of the ion exchange system may be heated stepwise. Even in a case where the metal-exchanged ion exchange resin is used, the increased temperaturecanefficientlyremoveahalide [inparticular, e.g., a C₄₋₁₅alkyl iodide (preferably, e.g., a C₅₋₁₀alkyl iodide) such as pentyl iodide, neopentyl iodide, or hexyl iodide] while preventing the loss of the metal.

The temperature (such as the inner temperature) of the ion exchange system may for example be about 17 to 100°C, preferably about 18 to 80°C (e.g., about 20 to 70°C), and more preferably about 30 to 65°C (e.g., about 40 to 60°C). The temperature of the ion exchange system may be continuously increased, in particular, may be preferably increased stepwise. For example, in the initial stage of the operation, the stream is preferably brought into contact with the ion exchange resin at a relatively low temperature (e.g., about 17 to 35°C) to maintain the amount of silver and/or mercury or suppress the outflow or loss of silver and/or mercury in a small amount for increasing the rate of effective utilization of the ion exchange resin, thereby a halide (e.g., an iodide) is removed with fully utilizing the ion exchange resin bed [that is, until the performance of the ion exchange resin reaches a breakthrough (or arrives at a break through capacity)]. Then, by increasing the temperature of the ion exchange system to a second-stage temperature (a higher temperature, for example, about 40 to 45°C), the length of the absorption band (bed) is shortened and the moving speed of the stream passing through the adsorption band is decreased. Specifically, it is preferred to improve the ion exchange efficiency, extend the resin life (which is the time reached a breakthrough), and increase the usage rate. These effects increase as the temperature of the adsorption system rises. At the same time, since the rise in the temperature increases a loss of silver and/or mercury, it is advantageous that the second-stage temperature is about 10°C (e.g., about 5 to 15°C, preferably about 7 to 13°C, and more preferably about 8 to 12°C) higher than the initial temperature. Further, by repeatedly increasing the temperature stepwise (e.g., a third-stage temperature, a fourth-stage temperature) in the same manner as the second temperature, the removal efficiency of the halogen compound, and the usage rate (usage efficiency) of the ion exchange resin, and silver and mercury can be significantly improved, while the deterioration of the ion exchange resin and the loss of silver and/or mercury are suppressed.

The flow rate of the carboxylic acid stream is not particularly limited to a specific one, and may be, e.g. , about 3 to 15 bed volumes/h, preferably about 5 to 12 bed volumes/h, and more preferably about 6 to 10 bed volumes/h for a column having a guard bed.

According to the process of the present invention, an impurity (e.g., a metal-containing impurity, a carbonyl impurity, and a halide) can be highly removed, and a high-purity product carboxylic acid (e.g., acetic acid) can be produced. The high-purity product carboxylic acid has significantly improved potassium permanganate test value and/or potassium bichromate test value. For example, the potassium permanganate test value can be improved up to not less than 120 minutes, which is required for the product standard, not less than 160 minutes (e.g., about 170 to 500 minutes), preferably not less than 180 minutes (e.g., about 190 to 450 minutes), and more preferably not less than 200 minutes (e.g., about 210 to 430 minutes). Moreover, the potassium bichromate test value can for example be improved up to not less than 120 minutes (e.g., about 130 to 300 minutes), preferably not less than 140 minutes (e.g., about 140 to 250 minutes), and more preferably not less than 145 minutes (e.g., about 145 to 200 minutes).

It is preferable that the product carboxylic acid satisfy at least one of the above range of the potassium permanganate test value and the above range of the potassium bichromate test value. In particular, it is preferable that both test values be within the above ranges.

### EXAMPLES

The following examples are intended to describe this invention in further detail and should by no means be interpreted as defining the scope of the invention.

### Comparative Example 1

A stirred-tank reactor (an autoclave equipped with a stirrer; material of reactor: HASTELLOY B-2 (registered trademark), nickel alloy, manufactured by Haynes International) containing a reaction liquid (10 L) was used for continuously producing acetic acid at a reaction pressure of 2.76 MPaG and a reaction temperature of 186.8°C. The composition of the reaction liquid was as follows: a water concentration of 3.9% by weight, a methyl acetate concentration of 2.5% by weight, a methyl iodide concentration of 11.9% by weight, a LiI concentration of 14.8% by weight, and a Rh concentration of 1670 ppm. Moreover, the gaseous phase in the reactor had a H₂ partial pressure of 0.050 MPa, and the STY (space time yield) of acetic acid was 27.8 mol/L·h.

The reaction liquid obtained from the reactor was subjected to the process flow (diagram, purification treatment) shown in Fig. 2 except that a second flash system (flash evaporator) was not used or operated. Then the obtained acetic acid product from the first distillation column was evaluated. That is, the reaction liquid was continuously fed to the middle (the middle in the height direction) of a first flash system (a flash evaporation tank equipped with no stirrer) for an adiabatic flashing. A fraction (stream) containing vaporized acetic acid was withdrawn from the top of the flash evaporation tank and fed to the 26th plate from the bottom of a distillation system [distillation column: packed column, a packing made of zirconium ("Techno-Pack" sold by Mitsui & Co., Ltd.), theoretical number of plates: 38, pressure of column top: 0.14 MPaG (gauge pressure), temperature of column top: 115°C]. An impuritywas separated from the top of the column, and a stream mainly containing acetic acid was collected from the first plate from the bottom of the column. The impurity-containing fraction separated from the top of the distillation column was then fed to a condenser and condensed (liquefied), and a portion of the resulting condensate was refluxed to the first plate from the top of the distillation column (returned to the distillation column by reflux), and the remainder was recycled to the reactor. The reflux ratio was 0.7. The fraction separated from the top of the distillation column contained an impurity, e.g. , hydrogen iodide, methyl iodide, methyl acetate, acetaldehyde, and water.

In this Comparative Example, the remainder of the condensate was recycled to the reactor without acetaldehyde (AD)-removingtreatment. Moreover, the stream containing acetic acid obtained from the distillation column was directly collected as a product acetic acid without treatment with an ion exchange resin or others, and the quality of the product acetic acid was evaluated.

### Example 1

In the same manner as in Comparative Example 1, the reaction and the purification treatment of acetic acid were continuously carried out except for the followings. In this Example, as shown in the diagram of Fig. 2, the stream containing acetic acid collected from the distillation column was fed through a side feed port of a flash evaporator [a flash evaporator equipped with an external circulation heating tube (in Fig. 2, the external circulation heating tube was omitted), atmospheric pressure, temperature: 118°C] to the inside of the evaporator. A stream containing acetic acid vaporized by the external circulation heating tube was withdrawn from the top of the evaporator and liquefied by a condenser. The liquefied stream containing acetic acid was directly collected as a product acetic acid without treatment with an ion exchange resin or others, and the quality of the product acetic acid was evaluated.

The results of Comparative Example 1 and Example 1 are shown in Table 1.

[Table 1]

**Table 1**

| Item | Comparative Example 1 | Example 1 |
|---|---|---|
| External appearance | Colorless and transparent, no floating matter | Colorless and transparent, no floating matter |
| Hue (Hazen color number) | 14 | 10 |
| Purity (% by weight) | 99.92 | 99.94 |
| Water (% by weight) | 0.035 | 0.021 |
| Potassium permanganate test (min.) | 150 | 200 |
| Formic acid (% by weight) | 0.0016 | 0.0016 |
| Aldehyde (% by weight) | 0.0063 | 0.0063 |
| Iron (ppm) | 0.91 | 0.15 |
| Acid wash color | 15 | 5 |
| Heavy metal (ppm) | <0.5 | <0.5 |
| Sulfate (ppm) | <0.5 | <0.5 |
| Chloride (ppm) | <0.5 | <0.5 |
| Potassium bichromate test (min.) | 150 | 150 or more |

### Comparative Example 2

The continuous reaction was carried out in the same manner as in Comparative Example 1 except that the reaction temperature was changed to 185. 7°C and that the proportions of the reaction components were suitably controlled. The composition of the reaction liquid was as follows : a water concentration of 2.3% by weight, a methyl acetate concentration of 5.4% by weight, a methyl iodide concentration of 12.1% by weight, a LiI concentration of 21.0% by weight, and a Rh concentration of 1900 ppm. Moreover, the gaseous phase in the reactor had a H₂ partial pressure of 0.004 MPa, and the STY of acetic acid was 26.6 mol/L·h.

The purification treatment was continuously carried out in the same manner as in Comparative Example 1 except that the reaction liquid obtained from the above-mentioned reactor was used.

### Example 2

In the same manner as in Comparative Example 2, the reaction and the purification treatment were continuously carried out except for the followings. In this Example, as shown in the diagram of Fig. 2, the stream containing acetic acid collected from the distillation column was fed through a side feed port of a flash evaporator [a flash evaporator equipped with an external circulation heating tube (in Fig. 2, the external circulation heating tube was omitted), atmospheric pressure, temperature: 118°C] to the inside of the evaporator. A stream containing acetic acid vaporized by the external circulation heating tube was withdrawn from the top of the evaporator and liquefied by a condenser. The liquefied stream containing acetic acid was directly collected as a product acetic acid without treatment with an ion exchange resin or others, and the quality of the product acetic acid was evaluated.

The results of Comparative Example 2 and Example 2 are shown in Table 2.

[Table 2]

**Table 2**

| Item | Comparative Example 2 | Example 2 |
|---|---|---|
| External appearance | Colorless and transparent, no floating matter | Colorless and transparent, no floating matter |
| Hue (Hazen color number) | 18 | 10 |
| Purity (% by weight) | 99.92 | 99.94 |
| Water (% by weight) | 0.037 | 0.023 |
| Potassium permanganate test (min.) | 200 | 240 or more |
| Formic acid (% by weight) | 0.0001 | 0.0001 |
| Aldehyde (% by weight) | 0.0067 | 0.0067 |
| Iron (ppm) | 1.1 | 0.18 |
| Acid wash color | 17 | 5 |
| Heavy metal (ppm) | <0.5 | <0.5 |
| Sulfate (ppm) | <0.5 | <0.5 |
| Chloride (ppm) | <0.5 | <0.5 |
| Potassium bichromate test (min.) | 130 | 150 |

### Comparative Example 3

The continuous reaction was carried out in the same manner as in Comparative Example 1 except that the reaction temperature was changed to 187. 6°C and that the proportions of the reaction components were suitably controlled. The composition of the reaction liquid was as follows : a water concentration of 4.2% by weight, a methyl acetate concentration of 2.1% by weight, a methyl iodide concentration of 8.9% by weight, a LiI concentration of 10.2% by weight, and a Rh concentration of 900 ppm. Moreover, the gaseous phase in the reactor had a H₂ partial pressure of 0.064 MPa, and the STY of acetic acid was 12.0 mol/L·h.

The reaction liquid obtained from the above-mentioned reactor was subjected to the process flow (diagram, purification treatment) shown in Fig. 3 except that a second flash system (flash evaporator) was not used or operated. Then the acetic acid product from the second distillation column was evaluated.

Specifically, the reaction liquidwas continuously fed to the middle (the middle in the height direction) of a first flash system (a flash evaporation tank equipped with no stirrer) for an adiabatic flashing. A fraction (stream) containing vaporized acetic acid was withdrawn from the top of the flash evaporation tank and fed to the second plate from the bottom of a first distillation system [first distillation column: plate column, theoretical number of plates: 10, pressure of column top: 0.14 MPaG (gauge pressure), temperature of column top: 115°C]. An impurity was separated from the top of the column, and a stream mainly containing acetic acid and water was collected from the 5th plate from the bottom of the column.

The fraction containing the impurity separated from the top of the first distillation column was then fed to a condenser and condensed (liquefied), and a portion of the resulting condensate was refluxed to the first plate from the top of the distillation column (returned to the distillation column by reflux), and the remainder was recycled to the reactor. The reflux ratio in the first distillation column was 0.4. The fraction separated from the top of the first distillation column contained an impurity, e.g., hydrogen iodide, methyl iodide, methyl acetate, and acetaldehyde.

The stream containing acetic acid and water from the first distillation column was then fed to the 16th plate from the bottom of a second distillation system [second distillation column: plate column, theoretical number of plates: 25, pressure of column top: 0.18 MPaG (gauge pressure), temperature of column top: 135°C]. A fraction containing water was separated from the top of the column, and a stream mainly containing acetic acid was collected from the bottom of the column.

The fraction containing water separated from the top of the second distillation column contained hydrogen iodide, methyl iodide, methyl acetate, acetaldehyde, and others, in very small quantities. The fraction from the top of the second distillation column was then fed to a condenser and condensed (liquefied), and a portion of the resulting condensate was refluxed to the first plate from the top of the second distillation column (returned to the distillation column by reflux), and the remainder was combined with the recycle stream obtained from the first distillation column, and the combined stream was recycled to the reactor. The reflux ratio in the second distillation column was 4.

The stream containing acetic acid obtained from the second distillation column was collected as a product acetic acid without treatment with an ion exchange resin or others, and the quality of the product acetic acid was evaluated.

In this Comparative Example, an offgas (omitted in Fig. 3) withdrawn from the condenser through the first distillation column was fed to the lower part of an acetaldehyde-removing column [aldehyde-absorption column: made of stainless steel (SUS316L), theoretical number of plates: 7, pressure of column top: 0.12 MPaG (gauge pressure)] and brought into countercurrent-contact with water (10°C), so that aldehyde (e.g., acetaldehyde) contained in the offgas was absorbed to water. The resulting aqueous solution was withdrawn as a waste liquid from the bottom of the aldehyde-absorption column. The amount of acetaldehyde contained in the withdrawn waste liquid was about 2.6 g/h.

### Example 3

In the same manner as in Comparative Example 3, the reaction and the purification treatment were continuously carried out except for the followings. In this Example, as shown in the diagram of Fig. 3, the stream containing acetic acid collected from the second distillation column was fed through a side feed port of a flash evaporator [flash evaporator equipped with an external circulation heating tube (in Fig. 3, the external circulation heating tube was omitted), atmospheric pressure, temperature: 118°C] to the inside of the evaporator. A stream containing acetic acid vaporized by the external circulation heating tube was withdrawn from the top of the evaporator and liquefied by a condenser. The liquefied stream containing acetic acid was directly collected as a product acetic acid without treatment with an ion exchange resin or others, and the quality of the product acetic acid was evaluated.

The results of Comparative Example 3 and Example 3 are shown in Table 3.

[Table 3]

**Table 3**

| Item | Comparative Example 3 | Example 3 |
|---|---|---|
| External appearance | Black extraneous substance is present. | Colorless and transparent, no floating matter |
| Hue (Hazen color number) | 10 | 3 |
| Purity (% by weight) | 99.95 | 99.96 |
| Water (% by weight) | 0.037 | 0.011 |
| Potassium permanganate test (min.) | 360 or more | 360 or more |
| Formic acid (% by weight) | 0.0048 | 0.0031 |
| Aldehyde (% by weight) | 0.0039 | 0.001 |
| Iron (ppm) | 1.0 | 0.02 |
| Acid wash color | 10 | 3 |
| Heavy metal (ppm) | <0.5 | <0.5 |
| Sulfate (ppm) | <0.5 | <0.5 |
| Chloride (ppm) | <0.5 | <0.5 |
| Potassium bichromate test (min.) | 40 | 150 |
| Iodine ion (ppb) | 34 | - |
| Methyl iodide (ppb) | 19 | - |
| Hexyl iodide (ppb) | 12 | - |

### Comparative Example 4

The continuous reaction was carried out in the same manner as in Comparative Example 3 except that the reaction temperature was changed to 187. 2°C and that the proportions of the reaction components were suitably controlled. The composition of the reaction liquid was as follows : a water concentration of 2.5% by weight, a methyl acetate concentration of 2.0% by weight, a methyl iodide concentration of 8.5% by weight, a LiI concentration of 13.5% by weight, and a Rh concentration of 870 ppm. Moreover, the gaseous phase in the reactor had a H₂ partial pressure of 0.063 MPa, and the STY of acetic acid was 12.0 mol/L·h.

### Example 4

In the same manner as in Comparative Example 4, the reaction and the purification treatment were continuously carried out except for the followings. In this Example, as shown in the diagram of Fig. 3, the stream containing acetic acid collected from the second distillation column was fed through a side feed port of a flash evaporator [flash evaporator equipped with an external circulation heating tube (in Fig. 3, the external circulation heating tube was omitted), atmospheric pressure, temperature: 118°C] to the inside of the evaporator. A stream containing acetic acid vaporized by the external circulation heating tube was withdrawn from the top of the evaporator and liquefied by a condenser. The liquefied stream containing acetic acid was directly collected as a product acetic acid without treatment with an ion exchange resin or others, and the quality of the product acetic acid was evaluated.

The results of Comparative Example 4 and Example 4 are shown in Table 4.

[Table 4]

**Table 4**

| Item | Comparative Example 4 | Example 4 |
|---|---|---|
| External appearance | Colorless and transparent, no floating matter | Colorless and transparent, no floating matter |
| Hue (Hazen color number) | 3 | 3 |
| Purity (% by weight) | 99.94 | 99.96 |
| Water (% by weight) | 0.018 | 0.011 |
| Potassium permanganate test (min.) | 230 | 290 |
| Formic acid (% by weight) | 0.0033 | 0.0035 |
| Aldehyde (% by weight) | 0.0008 | 0.0008 |
| Iron (ppm) | 0.12 | 0.01 |
| Acid wash color | 3 | 3 |
| Heavy metal (ppm) | <0.5 | <0.5 |
| Sulfate (ppm) | <0.5 | <0.5 |
| Chloride (ppm) | <0.5 | <0.5 |
| Potassium bichromate test (min.) | 120 | 140 |
| Iodine ion (ppb) | 59 | - |
| Methyl iodide (ppb) | 4 | - |
| Hexyl iodide (ppb) | 9 | - |

### Example 5

In the same manner as in Comparative Example 2, the reaction and the purification treatment were continuously carried out according to the diagram of Fig. 2 except for the followings. In this Example, differently from the diagram of Fig. 2, a stream containing acetic acid withdrawn as a bottom liquid from the bottom of the distillation column was collected in a glass flask (volume : 1 L) equipped with a stirrer without using a flash evaporator. A powdery activated carbon (KURARAY COAL activated carbon KW10-32, manufactured by Kuraray Chemical Co., Ltd.) was added to the flask at a rate of 10 g of the activated carbon relative to 0.5 L of the bottom liquid collected in the flask, and the mixture was stirred for one hour at a temperature of 25°C and a stirring rate (rotational speed) of 100 rpm. Then, the stirring was stopped. The activated carbon was precipitated, and a supernatant was rapidly sampled. In the same manner as in other Examples and Comparative Examples, the resulting sample was evaluated. The results are shown in Table 5 by comparison with the date of Comparative Example 2.

[Table 5]

**Table 5**

| Item | Comparative Example 2 | Example 5 |
|---|---|---|
| External appearance | Colorless and transparent, no floating matter | Colorless and transparent, no floating matter |
| Hue (Hazen color number) | 18 | 3 |
| Purity (% by weight) | 99.92 | 99.94 |
| Water (% by weight) | 0.037 | 0.037 |
| Potassium permanganate test (min.) | 200 | 240 or more |
| Formic acid (% by weight) | 0.0001 | 0.0001 |
| Aldehyde (% by weight) | 0.0067 | 0.0051 |
| Iron (ppm) | 1.1 | 0.2 |
| Acid wash color | 17 | 3 |
| Heavy metal (ppm) | <0.5 | <0.5 |
| Sulfate (ppm) | <0.5 | <0.5 |
| Chloride (ppm) | <0.5 | <0.5 |
| Potassium bichromate test (min.) | 130 | 170 |
| Iodine ion (ppb) | 60 | 12 |
| Methyl iodide (ppb) | 19 | 8 |
| Hexyl iodide (ppb) | 12 | 5 |

In Examples and Comparative Examples, each characteristic described in Table 1 to Table 5 was evaluated in accordance with the methods as defined by JIS (Japanese Industrial Standards).

### INDUSTRIAL APPLICABILITY

The present invention is useful as an industrial productionprocess of a carboxylic acid (e.g., acetic acid), particularly, a continuous production process of a carboxylic acid, for obtaining a highly purified carboxylic acid (high-purity carboxylic acid).

### DESCRIPTION OF REFERENCE NUMERALS

- 1: First flash system (flash distillation column)
- 5: Distillation system (distillation column)
- 8, 28, 38: Condenser
- 9a, 29a, 39a: Reflux line
- 9b, 29b, 39b: Withdrawing line
- 10: Second flash system (flash evaporator)
- 14, 20: Recycle line
- 21: Reaction system
- 25: First distillation column
- 35: Second distillation column

## Claims

1. A process for producing a carboxylic acid, comprising:
subjecting a carboxylic acid stream containing a metal-containing impurity to a first flash system to form a vaporized fraction,
distilling the vaporized fraction by a distillation system to separate into a stream mainly containing a carboxylic acid and a volatile fraction containing a high-volatile impurity, and
feeding the separated stream mainly containing the carboxylic acid to a second flash system or an adsorption system to collect a purified carboxylic acid.

2. A process according to claim 1, wherein the distillation system comprises at least one distillation column.

3. A process according to claim 1 or 2, wherein
the carboxylic acid stream is obtainable by allowing an alkanol to react with carbon monoxide in a reaction system in the presence of a metal catalyst and contains the metal-containing impurity and a carbonyl-group-containing impurity, and
the purified carboxylic acid from which the metal-containing impurity and the carbonyl-group-containing impurity have been removed is collected.

4. A process according to any one of claims 1 to 3, wherein
a purified acetic acid is produced from an acetic acid stream containing a metal-containing impurity and a carbonyl-group-containing impurity, the acetic acid stream being obtainable by allowing methanol to react with carbon monoxide in a reaction system in the presence of a rhodium catalyst, an alkali metal iodide as a catalyst stabilizer, methyl iodide as a co-catalyst and water, and
at least one member selected from the group consisting of the reaction system; the first flash system; the distillation system; a stream feed line for coupling the reaction system with the first flash system; a stream feed line for coupling the first flash system with the distillation system; and a stream feed line for coupling the distillation system with the second flash system or the adsorption system is made of a metal.

5. A process according to claim 3 or 4, wherein at least one member selected from the group consisting of the reaction system for allowing the alkanol to react with carbon monoxide; the first flash system; the distillation system; a stream feed line for coupling the reaction system with first flash system; a stream feed line for coupling the first flash system with the distillation system; and a stream feed line for coupling the distillation system with the second flash system or the adsorption system is made of an iron alloy, a nickel alloy, metal zirconium, a zirconium alloy, metal titanium, or a titanium alloy.

6. A process according to any one of claims 3 to 5, wherein
at least one member selected from the group consisting of the reaction system, the first flash system, the second flash system, and the distillation system is made of a nickel alloy, metal zirconium, or a zirconium alloy, and
at least one member selected from the group consisting of a stream feed line for coupling the reaction system with the first flash system, a stream feed line for coupling the first flash system with the distillation system, and a stream feed line for coupling the distillation system with the second flash system or the adsorption system is made of a stainless steel.

7. A process according to any one of claims 1 to 6, wherein the first flash system comprises a flash evaporation tank, and the second flash system comprises a flash evaporation tank or a flash evaporator.

8. A process according to any one of claims 1 to 7, wherein the stream containing the carboxylic acid from the distillation system is subjected to the second flash system under a condition in which a temperature is 30 to 210°C and a pressure is 3 to 1, 000 kPa for separating into a less-volatile impurity and the carboxylic acid.

9. A process according to any one of claims 1 to 7, wherein the stream mainly containing the carboxylic acid separated in the distillation system is brought into contact with an adsorbent, without an oxidation treatment of the stream, for adsorbing a less-volatile impurity contained in the stream to the adsorbent to separate the carboxylic acid from the less-volatile impurity.

10. A process according to any one of claims 1 to 9, wherein the metal-containing impurity comprises:
(i) at least one selected from the group consisting of a metal catalyst, a deactivated product thereof, a catalyst stabilizer, and a deactivated product thereof, and/or
(ii) an impurity produced by corrosion of at least one metallic member selected from the group consisting of the reaction system, the first flash system, and a stream feed line for coupling the reaction system with the first flash system.

11. A process according to any one of claims 3 to 10, which further comprises subjecting the fraction containing the high-volatile impurity separated in the distillation system to an aldehyde separation system to remove an aldehyde from the high-volatile impurity for recycling the high-volatile impurity to the reaction system.

12. A process according to any one of claims 1 to 11, which further comprises treating a stream containing the carboxylic acid from the second flash system or the adsorption system with an ion exchange resin for obtaining a further purified carboxylic acid, wherein the treatment with the ion exchange resin is carried out under a higher temperature.

13. A process according to any one of claims 1 to 12, the purified carboxylic acid has a potassium bichromate test value of not less than 140 minutes and a potassium permanganate test value of not less than 160 minutes.
